(19) Europäisches Patentamt
European Patent Office
Office européen des brevets

(11) EP 4 219 520 A1

(12) EUROPEAN PATENT APPLICATION
published in accordance with Art. 153(4) EPC

(43) Date of publication:
**02.08.2023 Bulletin 2023/31**

(21) Application number: **21872040.7**

(22) Date of filing: **19.08.2021**

(51) International Patent Classification (IPC):
***C07H 21/02*** *(2006.01)* ***C12N 15/09*** *(2006.01)*

(52) Cooperative Patent Classification (CPC):
**C07H 21/02; C12N 15/09;** Y02P 20/55

(86) International application number:
**PCT/JP2021/030423**

(87) International publication number:
**WO 2022/064908 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2020 JP 2020159460**

(71) Applicant: **SUMITOMO CHEMICAL COMPANY, LIMITED**
**Tokyo 103-6020 (JP)**

(72) Inventors:
- **MIYAGAWA, Takuya**
  **Oita-shi, Oita 870-0106 (JP)**
- **OKUMURA, Hideki**
  **Oita-shi, Oita 870-0106 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) METHOD FOR PRODUCING NUCLEIC ACID OLIGOMER

(57) The present invention provides an efficient process for preparing a nucleic acid oligomer, specifically a method for deprotecting a protecting group of hydroxy group in the nucleic acid oligomer effectively. The present invention provides also a process for preparing a nucleic acid oligomer represented by formula (4) which comprises contacting a nucleic acid oligomer represented by formula (3) with a fluoride ion under an atmosphere of an inert gas or inert gases containing 15 % or less of oxygen concentration (wherein each definition of the groups described in the formula (3) and the formula (4) are the same as those described in the Description).

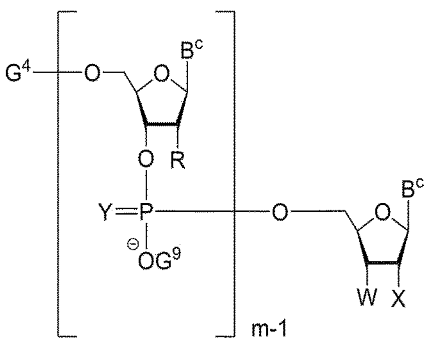
(3)

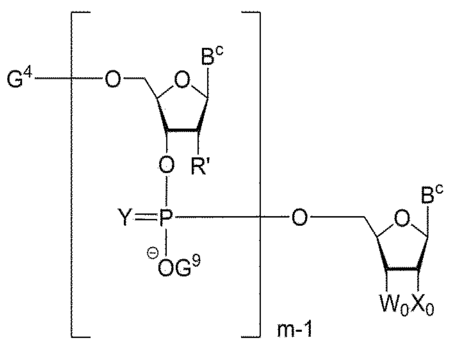
(4)

EP 4 219 520 A1

## Description

TECHNICAL FIELD

This application claims priority to and the benefit of Japanese Patent Application No. 2020-159460 filed September 24, 2020 according to the Paris Convention, the entire contents of which are incorporated herein by reference.

The present invention relates to a process for preparing a nucleic acid oligomer containing ribose, and in more details, relates to a deprotecting method of a protecting group of hydroxy group in a ribose which is contained in a nucleic acid oligomer.

BACKGROUND ART

In recent years, an attention in the application of nucleic acid oligomers to the medical field has been increased. Examples of the nucleic acid include an antisense nucleic acid, an aptamer, a ribozyme, and a nucleic acid which derives the RNA interference (RNAi) (such as siRNA) and the others, which are called a nucleic acid medicine.

A nucleic acid oligomer can be synthesized according to a solid phase synthesis, and in the solid phase synthesis, a phosphoramidite (hereinafter, referred to as "amidite") of nucleoside is used as a starting material. A nucleic acid oligomer which is synthesized by an elongation of a nucleic acid on a solid support is cut off from the solid support, and then in the nucleic acid oligomer containing ribose, a protecting group of hydroxy group at 2' position of the ribose is excluded by deprotection to prepare a desired nucleic acid oligomer. The purity of the nucleic acid oligomer which is synthesized by such a method is not necessarily a satisfactory one because the process is passed through multi-steps such as an elongation step of nucleic acid on solid support, a cutting off step from a solid support, and a deprotection step of each protecting group, which as a result, the synthesis is not efficient (see Patent Literatures 1 and 2).

CITATION LIST

PATENT LITERATURE

Patent Literature 1: WO 2006/022323 A1
Patent Literature 2: WO 2013/027843 A1

SUMMARY OF THE INVENTION

(PROBLEMS TO BE SOLVED BY INVENTION)

An object of the present invention is to provide an efficient process for preparing a nucleic acid oligomer.

(MEANS TO SOLVE PROBLEMS)

The present inventors have intensively studied to achieve the above object, and can find out that a fluoride ion can contact with a nucleic acid oligomer under an atmosphere of an inert gas or inert gases containing the oxygen concentration below a certain level to deprotect effectively a protecting group of hydroxy group in a ribose which is contained in the nucleic acid oligomer, and as a result, can provide an efficient process for preparing a nucleic acid oligomer.

The present invention has been completed on the basis of these findings, and encompasses the following aspects, but are not limited thereto.

[1] A process for preparing a nucleic acid oligomer represented by formula (4):

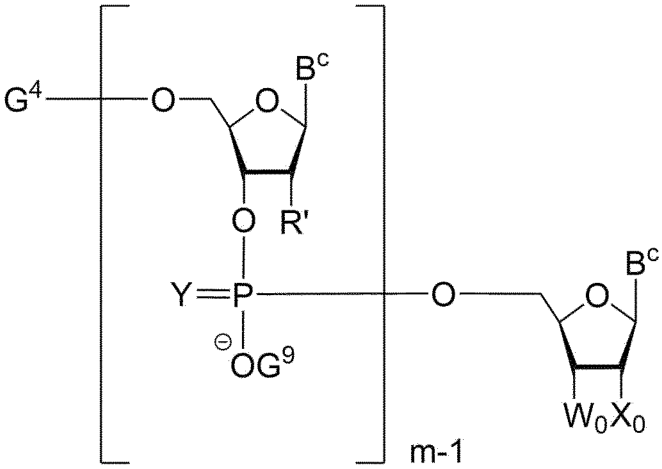

(4)

[wherein

R' is identical to or different from each other and each independently represents a hydroxy group, a hydrogen atom, a fluorine atom, a methoxy group, a 2-methoxyethyl group, or OQ' group,
Q' is identical to or different from each other and each independently represents a methylene group which is attached to a carbon atom at 4' position of ribose, an ethylene group which is attached to a carbon atom at 4' position of ribose, or an ethylidene group which is attached to a carbon atom at 4' position of ribose,
the definitions of substituents $G^4$, $G^5$, Y, $B^c$ and m of the formula (4) are the same as those defined in the below formula (3),
$W_0$ represents a hydroxy group,
$X_0$ are the same as those defined in the below R' group.
when m is an integer of 3 or more, the nucleic acid oligomer represented by formula (4) represents a nucleic acid oligomer which may be incorporated by a non-nucleotide linker instead of the number of p (with the proviso that p is a positive integer satisfying an equation: m-1 > p) of nucleotides between the respective nucleotides at 5' terminus and 3' terminus),
which comprises contacting a nucleic acid oligomer represented by formula (3):

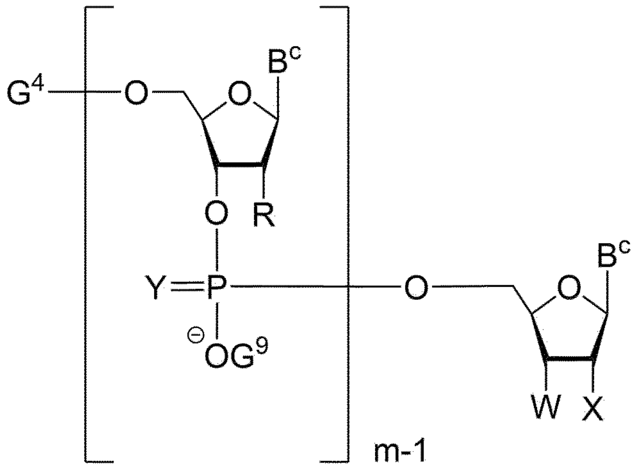

(3)

(wherein

$G^4$ represents a hydrogen atom or a protecting group of hydroxy group,
$G^9$ represents an ammonium ion, an alkylammonium ion, an alkali metal ion, a hydrogen ion, or a hydroxyalkylammonium ion,
$B^c$ represents a nucleobase, each of which is independently identical to or different from each other,
R are identical to or different from each other and each independently represents a hydrogen atom, a fluorine atom or OQ group,
Q is identical to or different from each other and each independently represents a tert-butyldimethylsilyl

group, a methyl group, a 2-methoxyethyl group, a methylene group which is attached to a carbon atom at 4' position of ribose, an ethylene group which is attached to a carbon atom at 4' position of ribose, an ethylidene group which is attached to a carbon atom at 4' position of ribose, or a protecting group represented by formula (1):

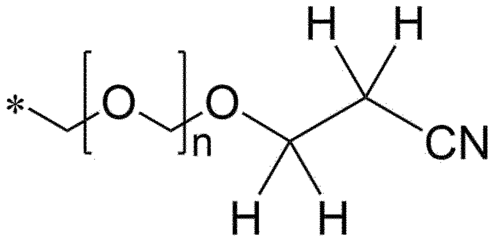 (1)

(wherein

a bond marked with * represents a bond to an oxygen atom of OQ group,
n is an integer of 0 or more),
Y are identical to or different from each other and each independently represents an oxygen atom or a sulfur atom,
m is an integer of 2 or more to 200 or less,
W and X are defined as either the following (a) or (b) :

(a) when W represents a hydroxy group, X is the same as defined as those of the above R group,
(b) when X represents a hydroxy group, W represents a OV group,

V represents a tert-butyldimethylsilyl group, or the group represented by the above formula (1).
with proviso that at least one group selected from the above R group, W group and X group represents a hydroxy group which is protected with a protecting group represented by the above formula (1), and when m is an integer of 3 or more, the nucleic acid oligomer represented by formula (3) represents a nucleic acid oligomer which may be incorporated by a non-nucleotide linker instead of the number of p (with the proviso that p is a positive integer satisfying an equation: m-1 > p) of nucleotides between the respective nucleotides at 5' terminus and 3' terminus)

with a fluoride ion under an atmosphere of an inert gas or inert gases containing 15 % or less of oxygen concentration (hereinafter, the method is referred to as "process for preparation of the present invention", or "Present process").

[2] The process according to [1] wherein n is 0 or 1 in formula (1).
[3] The process according to [1] wherein n is 0 in formula (1) .
[4] The process according to [1] wherein n is 1 in formula (1) .
[5] The process according to any one of [1] to [4] wherein the non-nucleotide linker is a linker comprising an amino acid backbone.
[6] The process according to [5] wherein the linker comprising an amino acid backbone is a linker having a structure selected from the following formula (A14-1), (A14-2) or (A14-3).

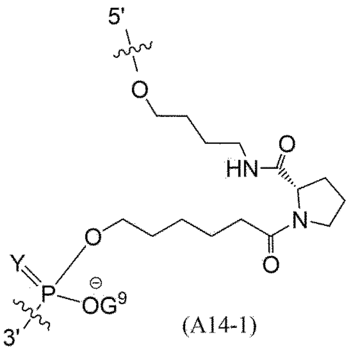

(A14-1) ,

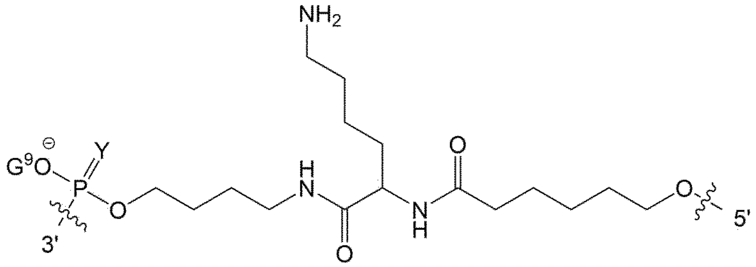

(A14-2)

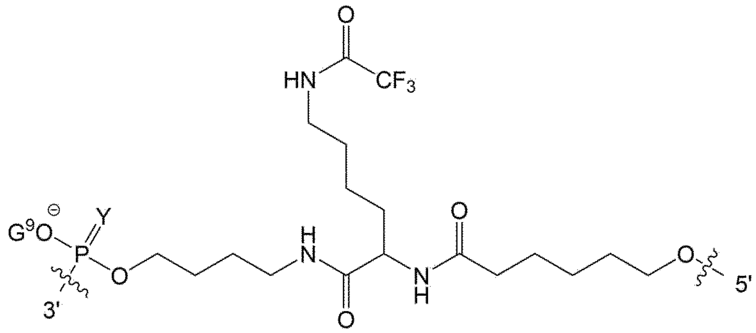

(A14-3)

(wherein a symbol of 5' and 3' represents a 5' terminus side and 3' terminus side respectively of the nucleic acid oligomer.).

[7] The process according to any one of [1] to [5] wherein W represents a hydroxy group, X represents a R group, $W_0$ represents a hydroxy group, and $X_0$ represents a R' group.

[8] The process according to any one of [1] to [7] wherein the fluoride ion source is tetraalkylammonium fluoride.

[9] The process according to any one of [1] to [8] wherein the tetraalkylammonium fluoride is tetra-n-butylammonium fluoride (TBAF).

[10] The process according to any one of [1] to [9] wherein the reaction is conducted under an atmosphere of an inert gas or inert gases containing 10 % or less of oxygen concentration.

[11] The process according to any one of [1] to [9] wherein the reaction is conducted under an atmosphere of an inert gas or inert gases containing 5 % or less of oxygen concentration.

[12] The process according to any one of [1] to [9] wherein the reaction is conducted under an atmosphere of an inert gas or inert gases containing 4 % or less of oxygen concentration.

[13] The process according to any one of [1] to [9] wherein the reaction is conducted under an atmosphere of an inert gas or inert gases containing 3 % or less of oxygen concentration.

[14] The process according to any one of [1] to [9] wherein the reaction is conducted under an atmosphere of an inert gas or inert gases containing 2 % or less of oxygen concentration.

[15] The process according to any one of [1] to [9] wherein the reaction is conducted under an atmosphere of an inert gas or inert gases containing 1 % or less of oxygen concentration.

[16] The process according to any one of [1] to [9] wherein the reaction is conducted under an atmosphere of an inert gas or inert gases containing 0 % of oxygen concentration.

[17] The process according to any one of [1] to [16] wherein the temperature in the reaction system in which the nucleic acid oligomer represented by formula (3) is contacted with the fluoride ion is 25 °C or less.

[18] The process according to any one of [1] to [16] wherein the temperature in the reaction system in which the nucleic acid oligomer represented by formula (3) is contacted with the fluoride ion is 20 °C or less.

[19] The process according to any one of [1] to [16] wherein the temperature in the reaction system in which the nucleic acid oligomer represented by formula (3) is contacted with the fluoride ion is 15 °C or less.

[20] The process according to any one of [1] to [16] wherein the temperature in the reaction system in which the nucleic acid oligomer represented by formula (3) is contacted with the fluoride ion is 10 °C or less.

[21] The process according to any one of [1] to [16] wherein the temperature in the reaction system in which the nucleic acid oligomer represented by formula (3) is contacted with the fluoride ion is 5 °C or less.

[22] The process according to any one of [1] to [16] wherein a duration required for contacting the total amount of

the fluoride ion with the nucleic acid oligomer represented by formula (3) is 30 minutes or more.

[23] The process according to any one of [1] to [16] wherein a duration required for contacting the total amount of the fluoride ion with the nucleic acid oligomer represented by formula (3) is 1 hour or more.

[24] The process according to any one of [1] to [23] wherein among the R group, the W group, and the X group in the nucleic acid oligomer represented by formula (3), the ratio of a protecting group represented by the above formula (1) is 10 % or more, and the nucleic acid chain length is 10 mer or more.

[25] The process according to any one of [1] to [23] wherein among the R group, the W group, and the X group in the nucleic acid oligomer represented by formula (3), the ratio of a protecting group represented by the above formula (1) is 20 % or more, and the nucleic acid chain length is 10 mer or more.

[26] The process according to any one of [1] to [23] wherein among the R group, the W group, and the X group in the nucleic acid oligomer represented by formula (3), the ratio of a protecting group represented by the above formula (1) is 30 % or more, and the nucleic acid chain length is 10 mer or more.

[27] The process according to any one of [1] to [23] wherein among the R group, the W group, and the X group in the nucleic acid oligomer represented by formula (3), the ratio of a protecting group represented by the above formula (1) is 40 % or more, and the nucleic acid chain length is 10 mer or more.

[28] The process according to any one of [1] to [23] wherein among the R group, the W group, and the X group in the nucleic acid oligomer represented by formula (3), the ratio of a protecting group represented by the above formula (1) is 50 % or more, and the nucleic acid chain length is 10 mer or more.

[29] The process according to any one of [1] to [23] wherein among the R group, the W group, and the X group in the nucleic acid oligomer represented by formula (3), the ratio of a protecting group represented by the above formula (1) is 60 % or more, and the nucleic acid chain length is 10 mer or more.

[30] The process according to any one of [1] to [23] wherein among the R group, the W group, and the X group in the nucleic acid oligomer represented by formula (3), the ratio of a protecting group represented by the above formula (1) is 70 % or more, and the nucleic acid chain length is 10 mer or more.

[31] The process according to any one of [1] to [23] wherein among the R group, the W group, and the X group in the nucleic acid oligomer represented by formula (3), the ratio of a protecting group represented by the above formula (1) is 80 % or more, and the nucleic acid chain length is 10 mer or more.

[32] The process according to any one of [1] to [23] wherein among the R group, the W group, and the X group in the nucleic acid oligomer represented by formula (3), the ratio of a protecting group represented by the above formula (1) is 90 % or more, and the nucleic acid chain length is 10 mer or more.

[33] The process according to any one of [1] to [23] wherein among the R group, the W group, and the X group in the nucleic acid oligomer represented by formula (3), the ratio of a protecting group represented by the above formula (1) is 95 % or more, and the nucleic acid chain length is 10 mer or more.

[34] The process according to any one of [1] to [23] wherein among the R group, the W group, and the X group in the nucleic acid oligomer represented by formula (3), the ratio of a protecting group represented by the above formula (1) is 100 %, and the nucleic acid chain length is 10 mer or more.

[35] The process according to any one of [1] to [34] wherein the nucleic acid chain length is 20 mer or more.

[36] The process according to any one of [1] to [34] wherein the nucleic acid chain length is 30 mer or more.

[37] The process according to any one of [1] to [34] wherein the nucleic acid chain length is 40 mer or more.

[38] The process according to any one of [1] to [34] wherein the nucleic acid chain length is 50 mer or more.

Effect of Invention

The present invention provides an effective process for preparing a nucleic acid oligomer. According to a process of the present invention, an improvement in a purity of a nucleic acid oligomer prepared can be expected.

MODE FOR CARRYING OUT THE INVENTION

It is described a process for preparing a nucleic acid oligomer represented by formula (4) which comprises contacting a nucleic acid oligomer represented by formula (3) with a fluoride ion under an atmosphere of an inert gas or inert gases containing 15 % or less of oxygen concentration to deprotect the protecting group represented by the formula (1).

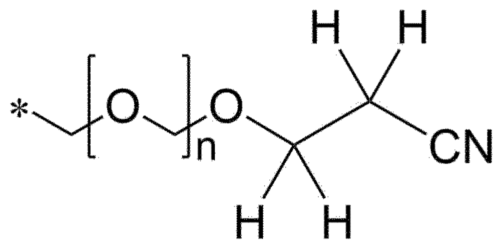

(1)

In the formula (1), n is any integer of 0 or more, more preferably an integer of 0 to 3, more preferably an integer of 0 to 2, still more preferably 0 or 1, and particularly preferably 1.

In the formula (3), at least one group selected from the R group, the W group and the X group represents a hydroxy group which is protected with a protecting group represented by the above formula (1). Among the R group, the W group, and the X group, the ratio of formula (1) may be 1 % or more, more preferably 5 % or more, more preferably 10 % or more, more preferably 20 % or more, more preferably 30 % or more, more preferably 40 % or more, more preferably 50 % or more, more preferably 60 % or more, more preferably 70 % or more, more preferably 80 % or more, more preferably 90 % or more, and still more preferably 95 % or more. Also the nucleic acid chain length to be synthesized is preferably 10 mer or more, more preferably 20 mer or more, more preferably 30 mer or more, more preferably 30 mer or more, and still more preferably 50 mer or more.

In the step of deprotecting a protecting group of hydroxy group represented by formula (1), examples of the fluoride ion source includes typically tetraalkylammonium fluoride.

Examples of the tetraalkylammonium fluoride include tetrabutylammonium fluoride, and tetramethylammonium fluoride and the others. A tetrabutylammonium fluoride (TBAF) is more preferably included.

The amount of the fluoride ion used is within a range of usually 1 to 1,000 mole(s), preferably 1 to 500 mole(s), more preferably 2 to 200 moles, and still more preferably 4 to 100 moles, as opposed to 1 mole of a protecting group removed.

In this step, organic solvent which is inactive to the reaction is usually used, and specific examples thereof include sulfoxide solvents, nitrile solvents, ether solvents, amide solvents, ketone solvents, aliphatic hydrocarbon solvents, ester solvents, aromatic solvents, or a mixed solvents of two or more of these solvents, and among these solvents, sulfoxide solvents are preferably included. Examples of the sulfoxide solvents include dimethyl sulfoxide and the others. Examples of the nitrile solvents include acetonitrile, propionitrile, and the others. Examples of the ether solvents include tetrahydrofuran and the others. Examples of the amide solvents include N-methyl-2-pyrrolidone and the others. Examples of the ketone solvents include acetone, methyl ethyl ketone, and the others. Examples of the aliphatic hydrocarbon solvents include hexane, heptane, and the others. Examples of the ester solvents include methyl acetate, ethyl acetate, and the others. Examples of the aromatic solvents include toluene, pyridine, and the others. Dimethyl sulfoxide, or a mixed solvent of dimethyl sulfoxide and acetonitrile is particularly preferably included.

The fluoride ion source, which is a reagent used in a step of deprotecting a protecting group of hydroxy group represented by formula (1), is used by dissolving it in a solvent, followed by usually dehydration of it. Examples of the dehydrating agents include molecular sheave, and sulfate salts, and the others, and a molecular sheave 4A is preferably used.

The amount of the solvent used is within a range of usually 5 to 8,000 L, preferably 50 to 2,000 L, and more preferably 100 to 1,600 L, as opposed to 1 mole of the nucleic acid oligomer which is supplied in a deprotecton step.

If necessary, it may be added with a capturing compound which is reacted with a compound represented by the following formula (2) as a by-product of this step to capture the compound. Examples of the capturing compound include nitroalkanes, alkylamines, amidines, thiols, thiol derivatives, or mixtures of two or more of these compounds. Examples of "nitroalkanes" include nitromethane. Examples of "alkylamines" include an straight chain alkylamine having 1 to 6 carbon atoms, and a cyclic amine having 1 to 8 carbon atoms. Specific examples thereof include methylamine, ethylamine, n-propylamine, n-butylamine, n-pentylamine, n-hexylamine, morpholine, and piperidine. Examples of "amidines" include benzamidine, and formamidine. Examples of "thiols" include a straight chain thiol having 1 to 6 carbon atoms. Specific examples of thiols include methanethiol, ethanethiol, 1-propanethiol, 1-butanethiol, 1-pentanethiol, and 1-hexanethiol. Examples of "thiol derivatives" include alcohols or ethers containing the straight chain alkyl thiol groups containing 1 to 6 carbon atoms wherein the straight chain alkyl thiol groups are identical to or different from each other. Specific examples of thiol derivatives include 2-mercaptoethanol, 4-mercapto-1-butanol, 6-mercapto-1-hexanol, mercaptomethyl ether, 2-mercaptoethyl ether, 3-mercaptopropyl ether, 4-mercaptobutyl ether, 5-mercaptopentyl ether, and 6-mercaptohexyl ether. As the capturing compound, nitromethane is more preferably used.

A compound represented by formula (2):

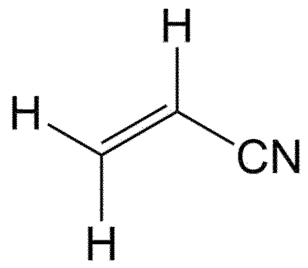 (2)

The used amount of the compound which captures the compound represented by formula (2) as a by-product can be within a range of 0.1 to 100.0 moles %, preferably 1.0 to 50.0 moles %, preferably 2.0 to 40.0 % moles, and more preferably 3.0 to 30.0 % moles, as opposed to the fluoride ion source which leaves a protecting group of hydroxy group represented by formula (1).

In the contacting reaction of the nucleic acid oligomer represented by formula (3) and the fluoride ion with each other, the fluoride ion may be added to the nucleic acid oligomer represented by formula (3). Or vice versa, the nucleic acid oligomer represented by formula (3) may be added to the fluoride ion, alternatively, both of the compounds may be added simultaneously with each other. The method of adding the fluoride ion to the nucleic acid oligomer represented by formula (3) is preferably included.

The duration required for adding the total amount of the fluoride ion to the nucleic acid oligomer represented by formula (3) to contact these compounds with each other is preferably an addition dropwise over 5 minutes or more, more preferably 10 minutes or more, more preferably 15 minutes or more, more preferably 30 minutes or more, and still more preferably 1 hour or more.

The addition is preferably an addition dropwise to a surface of the solution or to an inside of the solution containing the nucleic acid oligomer represented by formula (3) over 5 minutes or more, more preferably an addition dropwise over 10 minutes or more, more preferably an addition dropwise over 15 minutes or more, more preferably an addition dropwise over 30 minutes or more, and still more preferably an addition drowise over 1 hour or more.

When the fluoride ion is added to the nucleic acid oligomer represented by formula (3), the temperature of both or either of the solutions may be 80 °C or less, preferably the temperature of both thereof is 40 °C or less, preferably the temperature of both thereof is 35 °C or less, more preferably the temperature of both thereof is 30 °C or less, more preferably the temperature of both thereof is 25 °C or less, more preferably the temperature of both thereof is 20 °C or less, more preferably the temperature of both thereof is 15 °C or less, more preferably the temperature of both thereof is 10 °C or less, and still more preferably the temperature of both thereof is 5 °C or less.

After the completion of adding the fluoride ion to the nucleic acid oligomer represented by formula (3), the temperature of the reaction mixture may be kept for 1 minutes or more, preferably for 5 minutes or more, more preferably for 10 minutes or more, more preferably for 15 minutes or more, more preferably for 30 minutes or more, and still more preferably for 1 hour or more.

Further, after keeping the temperature, the temperature of the reaction mixture may be raised, may be raised to 5 °C or more to 80 °C or less, preferably the degree of the raised temperature is 10 °C or more to 40 °C or less, preferably the degree of the raised temperature is 10 °C or more to 35 °C or less, preferably the degree of the raised temperature is 15 °C or more to 35 °C or less, more preferably the degree of the raised temperature is 20 °C or more to 35 °C or less, and still more preferably the raised temperature is 25 °C or more to 35 °C or less.

Further, after raising the temperature, the period of deprotection reaction may be varied depending on the kind of the deprotecting agent used, or the reaction temperature, the period is within a range of usually 1 to 100 hour(s), preferably 1 to 24 hour(s), more preferably 2 to 12 hours, and still preferably 3 to 6 hours.

Here the fluoride ion may be added at any timing.

An atmosphere of an inert gas or inert gases containing 15 % or less of the oxygen concentration may be adjusted by preparing the inert gas or inert gases containing the above prescribed concentration or less of oxygen concentration, followed by supplying the gas or gases to the reaction system, and measuring and confirming that the oxygen concentration in the gas phase is within the above prescribed concentration range. Specifically, the oxygen concentration can be adjusted by the following methods, and for example, a high purity of inert gas (such as argon or nitrogen) or inert gases, or an inert gas or inert gases having the oxygen concentration adjusted to the prescribed concentration, is flowed in a gas phase of the reaction system, or alternatively, the gas or gases atmosphere of the reaction system is substituted with the above inert gas or inert gases, or the inert gas or inert gases having the concentration adjusted.

Examples of the inert gas or inert gases used in the process of the present invention include nitrogen gas, argon gas, helium gas, carbon dioxide, which are not limited thereto. Nitrogen gas or argon gas is preferably included.

The substitution method for reaction system atmosphere may be a substitution under reduced pressure, a

substitution under pressurized pressure, a flow substitution, a substitution using a bubbling, or a substitution using a freeze degassing, and an ultrasonic wave or a heat may be applied while these substitution methods are conducted. More preferable method include a flow substitution and a substitution under reduced pressure.

The oxygen concentration is preferably 15 % or less, more preferably 10 % or less, more preferably 5 % or less, more preferably 4 % or less, more preferably 3 % or less, more preferably 2 % or less, more preferably 1 % or less, and still more preferably 0 %.

Though a stirring procedure of the reaction system is not essenatial at the leaving reaction of the protecting group, the stirring is usually conducted within a range of 0.0 to 0.5 $kW/m^3$ of a stirring power Pv, and the stirring with 0.1 to 0.3 $kW/m^3$ of Pv is preferably included.

As the measures for separation and purification of the nucleic acid oligomer produced after the reaction from the reaction mixture, conventional methods can be adopted, and for example, using the measures such as extraction, concentration, neutralization, filtration, centrifugation, recrystalization, silicagel column chromatography, thin layer chromatography, reverse-phase column chromatography, ion exchange column chromatography, gel permeation column chromatography, hydrophobic interaction chromatography, hydrophilic interaction chromatography, precipitation (such as precipitation of nucleic acid oligomer using ethanol, isopropanol, methanol, or polyethylene glycol), dialysis, and ultrafiltration can deprotect a protection with a protecting group of hydroxy group at 2' position or 3' position represented by formula (1). The purified nucleic acid oligomer can be isolated. The isolated nucleic acid oligomer can be usually obtained as a nucleic acid oligomer having a hydroxy group at its 5' terminus protected.

The reaction in which a protecting group represented by formula (1) is deprotected from a nucleic acid oligomer represented by formula (3) to obtain a nucleic acid oligomer represented by the following formula (4) is described below (Scheme 1).

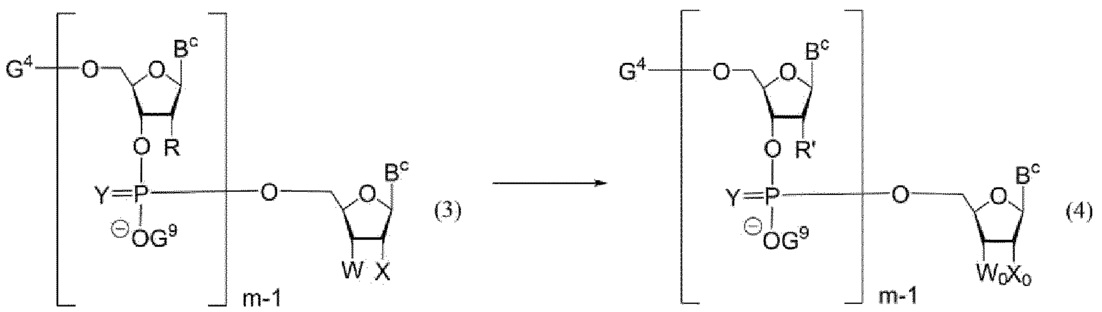

Scheme 1

(wherein

G4 represents a hydrogen atom or a protecting group of hydroxy group,
G9 represents an ammonium ion, an alkyl ammonium ion, an alkali metal ion, a hydrogen atom, or a hydroxyalkyl ammonium ion.
$B^c$ represents a nucleobase, each of which is independently identical to or different from each other,
R are identical to or different from each other and each independently represents a hydrogen atom, a fluorine atom or OQ group,
Q is identical to or different from each other and each independently represents a tert-butyldimethylsilyl group, a methyl group, a 2-methoxyethyl group, a methylene group which is attached to a carbon atom at 4' position of ribose, an ethylene group which is attached to a carbon atom at 4' position of ribose, an ethylidene group which is attached to a carbon atom at 4' position of ribose, or a protecting group represented by formula (1):

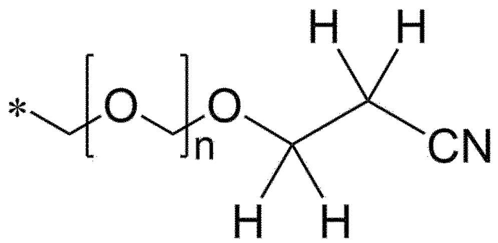

(1)

(wherein

a bond marked with * represents a bond to an oxygen atom of OQ group,
n is an integer of 0 or more),
Y are identical to or different from each other and each independently represents an oxygen atom or a sulfur atom,
m is an integer of 2 or more to 200 or less,
W and X are defined as either the following (a) or (b) :

(a) when W represents a hydroxy group, X is the same as defined as those of the above R group,
(b) when X represents a hydroxy group, W represents a OV group,

V represents a tert-butyldimethylsilyl group, or the group represented by the above formula (1).
with proviso that at least one group selected from the above R group, W group and X group represents a hydroxy group which is protected with a protecting group represented by the above formula (1), and
when m is an integer of 3 or more, the nucleic acid oligomer represented by formula (3) represents a nucleic acid oligomer which may be incorporated by a non-nucleotide linker instead of the number of p (with the proviso that p is a positive integer satisfying an equation: m-1 > p) of nucleotides between the respective nucleotides at 5' terminus and 3' terminus).

In the formula (3) or the formula (4), when R represents a OQ group, and R' represents a OQ' group, a structure of a ribose represents the following formula (LNA-1), (LNA-2) or (LNA-3).

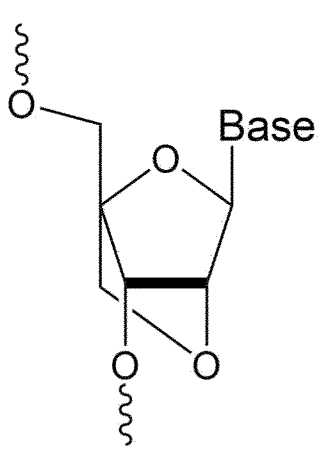

LNA-1

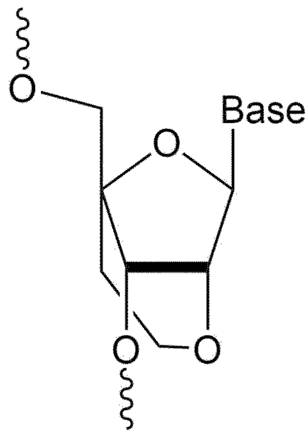

LNA-2

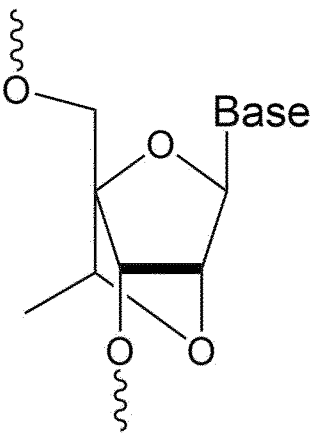

LNA-3

(wherein Base represents a nucleobase.).

As examples of the nucleoside (such as ribose, and deoxyribose) contained in the nucleic acid oligomer used in the present invention, DNA, RNA, 2'-O-MOE (2'-O-methoxyethyl), 2'-O-Me, 2'-F RNA, and the above LNA are exemplified, and the above nucleoside is not limited thereto.

The nucleic acid oligomer represented by formula (3) can be obtained, for example, by cutting out a nucleic acid oligomer represented by formula (5) which is prepared by a solid phase synthesis from a solid support, as shown in Scheme 2.

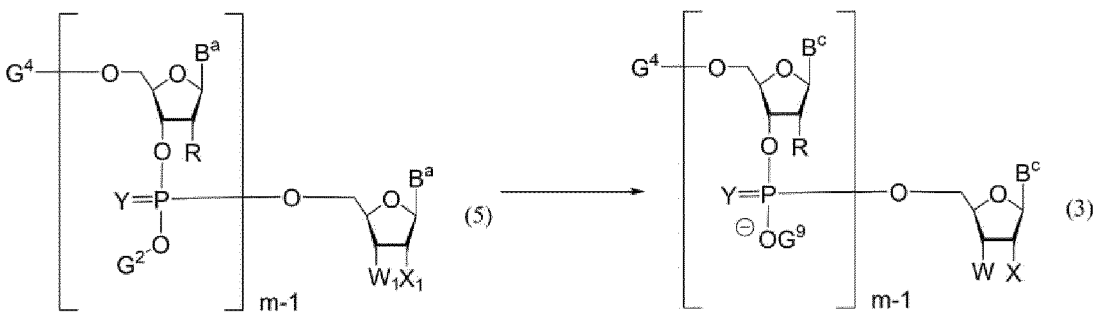
(5)
(3)

Scheme 2

The nucleic acid oligomer of formula (5) which is synthesized on a solid support is explained.

In the formula,

the substituent group $B^a$ represents a nucleobase which may be protected, each is independently identical to or different from each other,
$G^4$ and Y are the same as those defined in the above formula (3),
$G^2$ represents a protecting group of a phosphoric acid, each is independently identical to or different from to each other,
when $X_1$ represents OZ, $W_1$ represents OV group,
V represents a tert-butyldimethylsilyl group, or the above group represented by formula (1).
when $X_1$ represents a R group, $W_1$ represents a group represented by OZ.
Z represents a group consisting of a solid support, and a connection group connecting the solid support and the oxygen atom of a hydroxy group at 2' position or 3' position of a ribose at 3' terminus of the nucleic acid oligomer.

More specifically, Z represents a structure represented by the following formula (6):

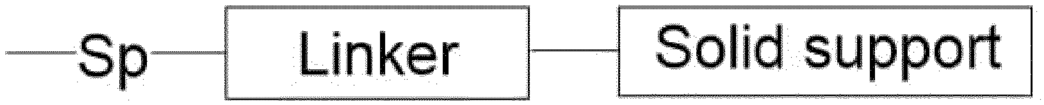


(6)

In formula (6), Sp represents a spacer.

The Spacer (Sp) is exemplified by a group having a structure represented by the following formula (7).

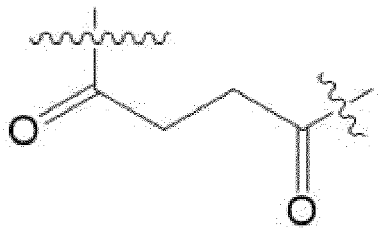

(7)

The Linker may be any structure represented by the following formula (8-1), (8-2), (8-3), (8-4), (8-5), (8-6), (8-7), or (8-8).

Examples of the solid support include an inorganic porous support, and organic resin support, and the others. Examples of the inorganic porous support include Controller pore Glass (CPG) and zeolite. Examples of the organic porous support include a support composed of polystyrene.

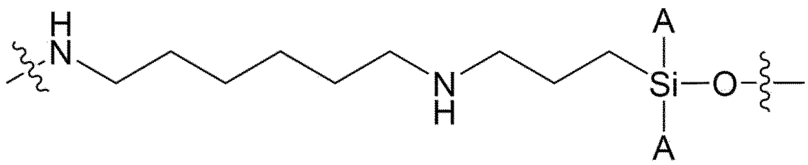

(8-1)

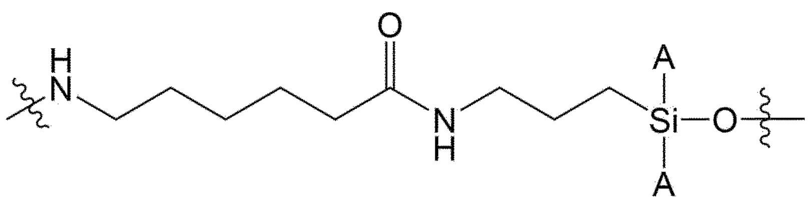

(8-2)

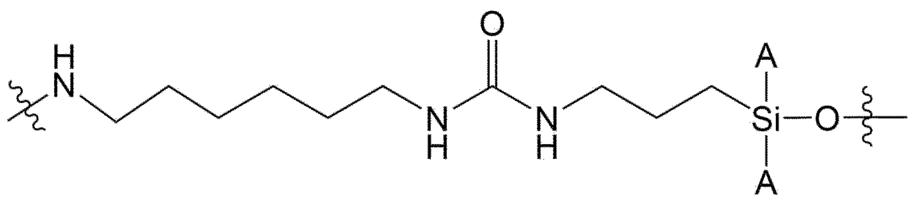
(8-3)

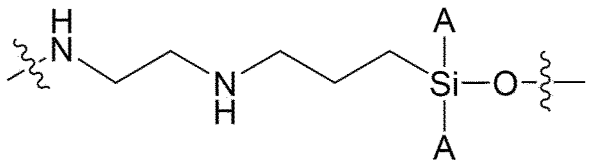
(8-4)

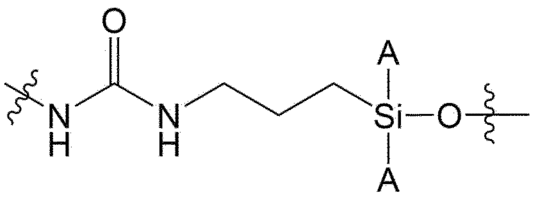
(8-5)

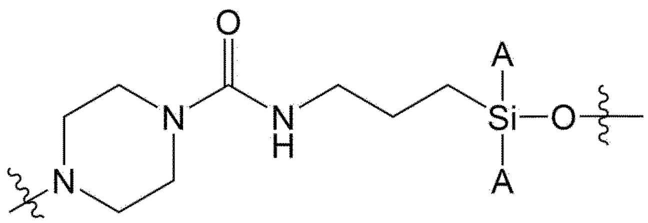
(8-6)

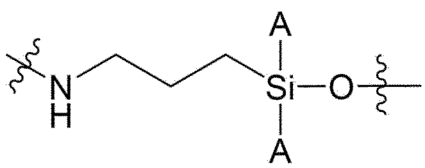
(8-7)

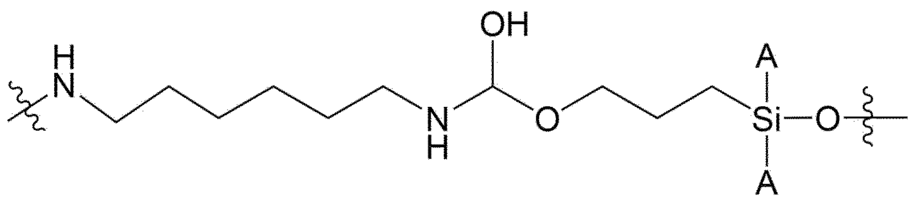
(8-8)

(wherein A may each independently represent a hydroxy group, an alkoxy group, or an alkyl group. Examples of the alkoxy group include a methoxy group and an ethoxy group. Examples of the alkyl group include a methyl group, an ethyl group, an isopropyl group, and a n-propyl group. Si represents a binding to an oxygen atom of a hydroxy group in a support surface.)

$G^4$ represents a hydrogen atom or a protecting group of hydroxy group, and when it represents a protecting group, it is the same as defined in $G^1$. When $G^4$ is deprotected, it is a hydrogen atom, and the nucleotide compound in the case is also provided in a series of steps for nucleic acid elongation reaction.

$G^9$ represents an ammonium ion, an alkyl ammonium ion, an alkali metal ion, a hydrogen atom, or a hydroxyalkyl ammonium ion. As alkyl ammonium ion, specific examples of alkyl part include methyl, ethyl, n-propyl, isopropyl, n-butyl, dibutyl, isobutyl, tert-butyl, n-pentyl, isopentyl, and hexyl, and specific examples thereof include diethyl ammonium ion, triethyl ammonium ion, tetrabutyl ammonium ion, hexyl ammonium ion, and dibutyl ammonium ion, and the others.

Examples of alkali metal ion include sodium ion and lithium ion. Also as hydroxy alkyl ammonium ion, specific examples of hydroxyalkyl part thereof include hydroxymethyl, hydroxyethyl, hydroxy-n-propyl, hydroxy isopropyl, hydroxy-n-butyl, and tris hydroxy methyl, and more specific examples of hydroxyalkyl ammonium ion include tris hydroxymethyl ammonium ion and the others.

The compound represented by the above formula (5) is prepared according to an amidite method by using an amidite compound represented by the following formula (A13).

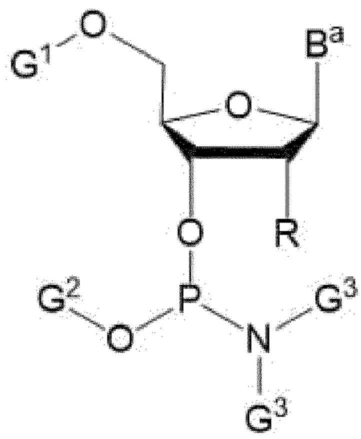

(A13)

(wherein

R represents a hydrogen atom, a fluorine atom, or OQ group,
Q represents a tert-butyldimethylsilyl group, a methyl group, a 2-methoxyethyl group, a methylene group which is attached to a carbon atom at 4' position, an ethylene group which is attached to a carbon atom at 4' position, an ethylidene group which is attached to a carbon atom at 4' position, or a protecting group represented by the above formula (1).
$B^a$ represents a nucleobase which may be optionally protected,
$G^1$ presents a protecting group of hydroxy group,
$G^2$ represents a protecting group of a phosphoric acid, and
$G^3$ represents an alkyl group, or a cyclic structure wherein both of $G^3$ may bind to each other at the respective terminus).

$B^a$ represents a nucleobase represented by $B^c$, or a nucleobase in which the nucleobase is protected with a protecting group.

Examples of the nucleobase as $B^a$ include adenine, cytosine, guanine, uracil, thymine, 5-methyl cytosine, pseudo uracil, 1-methyl pseudo uracil, and the others. Also the nucleic acid base may be optionally substituted with substituent(s). Examples of the substituent include a halogen atom (such as fluoro group, chloro group, bromo group, and iodo group), an acyl group (such as acetyl group), alkyl group (such as methyl group and ethyl group), arylalkyl group (such as benzyl group), alkoxy group (such as methoxy group), alkoxyalkyl group (such as methoxyethyl group), cyanoalkyl group (such as cyanoethyl group), hydroxy group, hydroxyalkyl group, acyloxymethyl group, amino group, monoalkylamino group, dialkylamino group, carboxy group, cyano group, and nitro group, as well as combinations of two or more of these substituents.

In the case where a nucleic acid base contains an exocyclic amino group, the protecting group of the amino group is not particularly limited, and the protecting group used in a publicly known nucleic acid chemistry field may be used, and examples of the protecting group include benzoyl group, 4-methoxybenzoyl group, acetyl group, propionyl group, butyryl group, isobutyryl group, phenylacetyl group, phenoxyacetyl group, 4-tert-butylphenoxyacetyl group, 4-isopropylphenoxyacetyl group, and (dimethylamino)methylene group, as well as combinations of two or more of these protecting groups.

$B^a$ represents more specifically any groups indicated below.

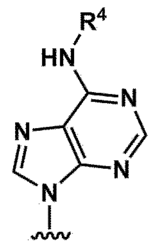
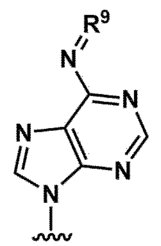

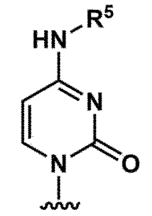
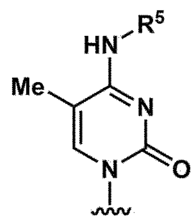

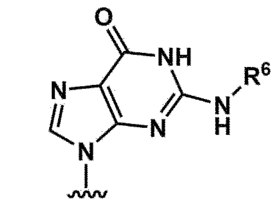
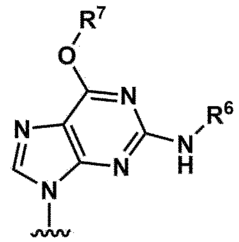
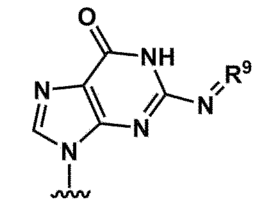
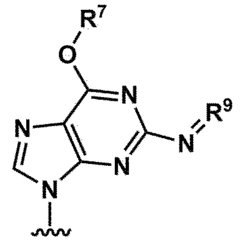

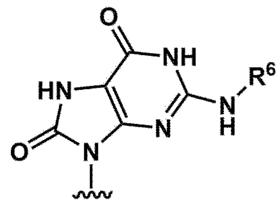
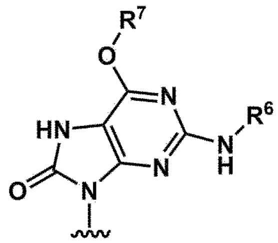
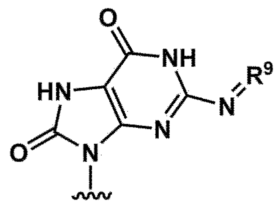
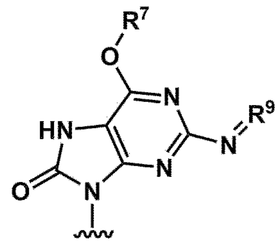

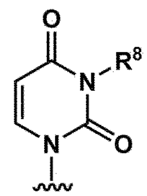
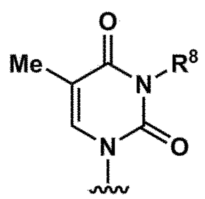
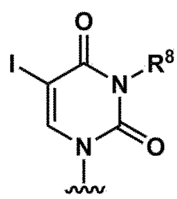
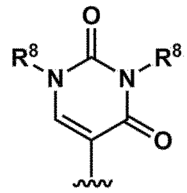

{wherein

$R^4$ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
$R^5$ represents a hydrogen atom, an acetyl group, an isobutyryl group, or a benzoyl group,
$R^6$ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
$R^7$ represents a 2-cyanoethyl group,
$R^8$ represents a hydrogen atom, a methyl group, a benzoyl group, a 4-methoxybenzoyl group, or a 4-methylbenzoyl group, and
$R^9$ represents a dimethylaminomethylene group.)

$G^1$ can be used without any particular limitation as long as it can function as a protecting group, and a publicly known protecting group used for the amidite compound can be used widely.

$G^1$ represents preferably the following groups.

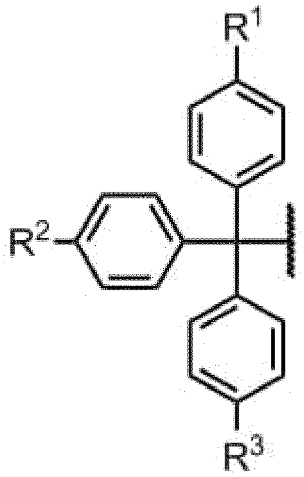

(wherein $R^1$, $R^2$ and $R^3$ are identical to or different from each other, and each independently represents a hydrogen atom or an alkoxy group.)

One of $R^1$, $R^2$ and $R^3$ represents a hydrogen atom, and the remaining two thereof are identical to or different from each other (preferably identical) and represents preferably an alkoxy group, and as an alkoxy group, a methoxy group is particularly preferred.

$G^2$ can be used without any particular limitation as long as it can function as a protecting group, and a publicly known protecting group used for an amidite compound can be widely used. Examples of $G^2$ include an alkyl group, an alkenyl group, an alkynyl group, a cycloalkyl group, a haloalkyl group, an aryl group, a heteroaryl group, an arylalkyl group, a cycloalkenyl alkyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri- alkylsilyloxyalkyl group, and the others, and these groups may be optionally substituted with one or more electron-withdrawing group.

$G^2$ represents preferably an alkyl group substituted with electron-attracting group. Examples of the electron-withdrawing group include a cyano group, a nitro group, an alkylsulfonyl group, a halogen atom, an arylsulfonyl group, a trihalomethyl group, a trialkylamino group, and the others, and preferably a cyano group.

Particularly preferable example of $G^2$ include the following groups.

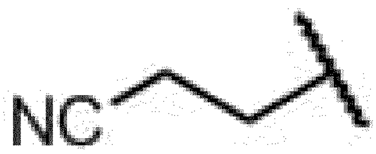

For $G^3$, two $G^3$ may be combined with each other to form a cyclic structure. Preferably, both $G^3$ are an isopropyl group.

The alkyl group as the definitions of the above $R^1$, $R^2$, $R^3$ and $G^2$ may be a straight chain or a branched chain, and preferably include an alkyl group containing 1 to 12 carbon atoms, and more preferably an alkyl group containing 1 to 6 carbon atoms. Specific examples of alkyl group include a methyl group, an ethyl group, a n-propyl group, an isopropyl group, a n-butyl group, an isobutyl group, a tert-butyl group, a n-pentyl group, an isopentyl group, and a hexyl group. An alkyl group part composed of the alkoxy group in the definition for above substituents has the same definition as that described in the definition of alkyl group described here.

As used herein, a nucleobase represents a group having a natural type or a non-natural type of nucleobase backbone. The above nucleobase encompasses also modified forms having the natural type or the non-natural type of nucleobase backbone modified. As a nucleobase represented by $B^c$, specific examples of the nucleobase are exemplified by the following structures.

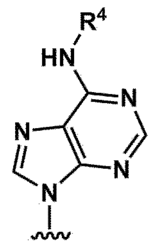
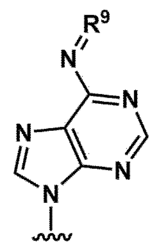

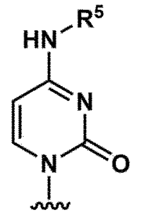
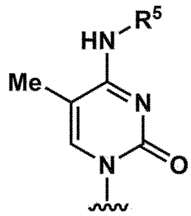

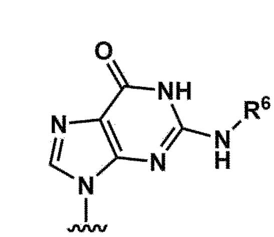
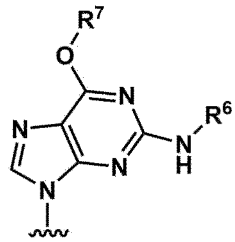
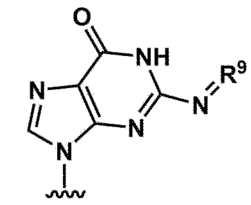
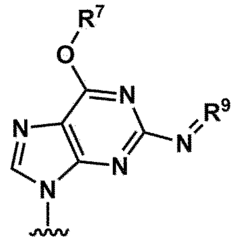

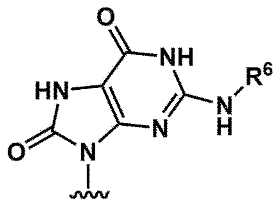
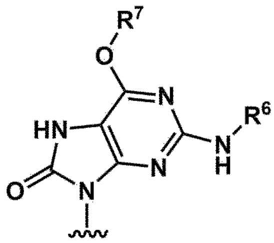
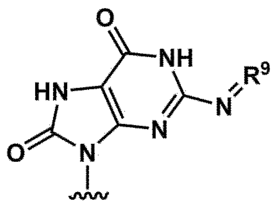
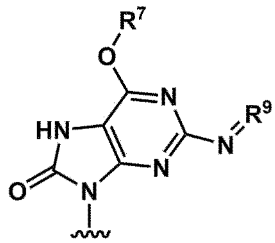

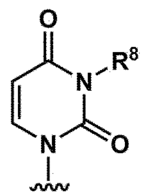
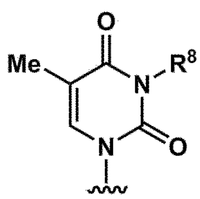
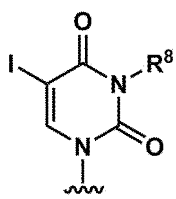
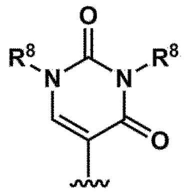

(wherein

$R^{4'}$ represents a hydrogen atom, or a methyl group,
$R^{5'}$ represents a hydrogen atom, or an acetyl group,
$R^{6'}$ represents a hydrogen atom,
$R^{8'}$ represents a hydrogen atom, or a methyl group.)

A non-nucleotide linker, which may be incorporated instead of the number of p (with the proviso that p is a positive integer satisfying an equation: m-1 > p) of nucleotides between the nucleotides at 5' terminus and 3' terminus, is described.

As non-nucleotide linker, a linker composed of amino acid backbone (for example, a linker composed of amino acid backbone as described in JP 5157168 B2 or JP 5554881 B2) is exemplified. Specifically, as a non-limiting example, a linker represented by formula (A14-1), (A14-2) or (A14-3) (for exmaple, as described in JP 5555346 B2 or JP 5876890 B2) is exemplified. In addition to these linkers, a particular linker as described in WO 2012/005368 A1, WO 2018/182008

or WO 2019/074110 is exemplified.

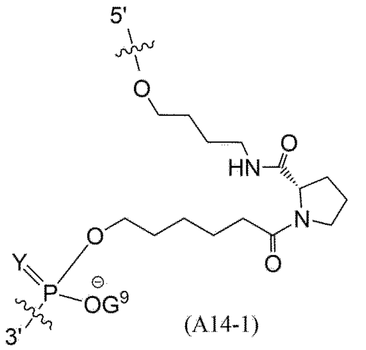

(A14-1) ,

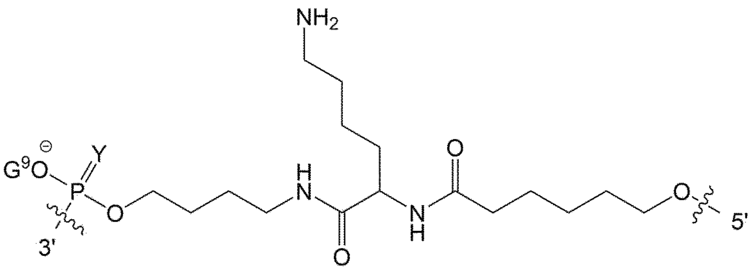

(A14-2)

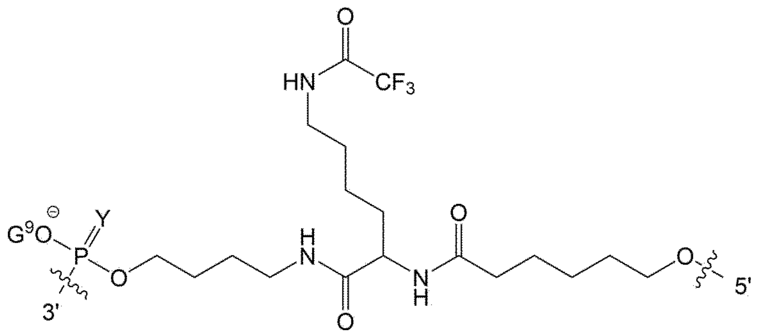

(A14-3)

A nucleotide and an amidite wherein a R group in formula (3) and a R' group in formula (4) are substituents other than a hydroxyl group, can also be prepared from nucleosides which are synthesized according to publicly known methods described in JP 3745226 B2 and on the others, or WO 2001/053528 A1, JP 2014-221817 A or publicly known methods referred to in these documents. Further, they can be prepared by using a commercially available compound in line with the method described in the below-mentioned Examples or methods with appropriate modifications to these methods.

Cutting off of nucleic acid oligomer (hereinafter, referred to as "oligonucleotide") from a solid support

The cutting off step was conducted by using concentrated aqueous ammonia water as a cutting off agent on a nucleic acid oligomer having a desirable chain length.

In a phosphoramidite method, an elongation reaction of nucleic acid is conducted by repeating each step such as a deprotection step, a condensation step and an oxidation step according to a generally known method (for example, the method described in the above JP 5157168 B2 or JP 5554881 B2).

(Nucleic acid elongation reaction)

As used herein, "nucleic acid elongation reaction" means that a reaction for elongating oligonucleotide by attaching nucleotide sequentially through phosphodiester bond. The nucleic acid elongation reaction can be carried out according to the procedures of general phosphoramidite method. The nucleic acid elongation reaction may be carried out with a nucleic acid automatic synthesizer and the others which applies a phosphoramidite method.

The chain length of a nucleic acid oligomer may be, for example, 2 to 200 mer, and 10 to 150 mer, and 15 to 110 mer.

A 5' deprotection step is a step where a protecting group of a 5' hydroxyl group at RNA chain terminus which is supported on the solid support. As a general protecting group, 4,4'-dimethoxytrityl group (DMTr group), 4-monomethoxytrityl group, and 4,4',4"-trimethoxytrityl group are used. A deprotection reaction can be carried out by using an acid. Examples of the acid for deprotection reaction include trifluoroacetic acid, dichloroacetic acid, trifluoroethanesulfonic acid, trichloroacetic acid, methanesulfonic acid, hydrochloric acid, acetic acid, p-toluenesulfonic acid, and the others.

The condensation step is a reaction where a nucleoside phosphoramidite represented by the following formula

(A13) is attached to a 5' hydroxyl group at oligonucleotide chain terminus deprotected by the above deprotection step. As the phosphoramidite to be used in the nucleic acid elongation, an amidite compound represented by formula (A13) or (A12) is used. Also, as another available phosphoramidite, 2'-OMe, 2'-F, 2'-O-tert-butyldimetylsilyl group, 2'-O-methoxyethyl group, 2'-H, 2'-fluro-2'-deoxy-β-D-arabinofuranosyl and the others are included. As the above nucleoside phosphoramidite, those where 5' hydroxyl group is protected with a protecting group (for example, DMTr group) are used. The condensation step can be carried out by using an activator which activates the above-mentioned nucleotide phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), and 5-(bis-3,5-trifluoromethylphenyl)-1H-tetrazole, and the others.

After the condensation step, as necessary, the unreacted 5' hydroxyl group may be capped. The capping reaction can be carried out by using publicly known capping solution such as acetic anhydride-tetrahydrofuran solution, and phenoxy acetic anhydride / N-methyl imidazole solution.

The oxidation step is a step for converting a phosphite group which is formed by the above condensation step into a phosphate group or a thiophosphate group. This step is a reaction of converting a trivalent phosphorus into a pentavalent phosphorus using an oxidizing agent, which can be carried out by reacting an oxidizing agent with oligonucleic acid derivatives supported on a solid support.

When a phosphite group is converted into a phosphate group, as "oxidizing agent", for example, an iodine, a peracid such as tert-butyl hydroperoxide and hydrogen peroxide, (1S)-(+)-(10-camphorsulfonyl)-oxazolidine (CSO), or a mixture of two or more of these compounds can be used. The oxidizing agent can be used by diluting it with an appropriate solvent so as to adjust to 0.005 to 2 M. The solvents to be used in the reaction are not particularly limited as long as they do not disturb the reaction, and include pyridine, THF, water, acetonitrile, or any mixed solvents of two or more of these solvents. For example, iodine/water/pyridine/acetonitrile, or iodine/water/pyridine, or iodine/water/pyridine/acetonitrile/NMI, or iodine/water/pyridine/THF, or iodine/water/pyridine/THF/NMI, or CSO/acetonitrile, or iodine/pyridine-acetic acid or peracid (tert-butyl hydroperoxide/methylene chloride) can be used.

When a phosphite triester group is converted into a thiophosphate group, as "oxidizing agent", for example, a sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethylidene)amino]-3H-1,2,4-dithiazoline-3-thione (DDTT), and phenylacetyldisulfide (PADS) can be used. The oxidizing agent can be used by diluting it with an appropriate solvent so as to adjust to 0.01 to 2 M. The solvents to be used in the reaction are not particularly limited as long as they do not involve the reaction, and include, for example, dichloromethane, acetonitrile, pyridine, or any mixed solvents of these solvents. The oxidation step may be carried out after the above mentioned capping procedure, or vice versa, the capping procedure may be carried after the oxidation step, and the order of the procedures are not limited.

In the step of deprotecting a protecting group of a phosphorus group, when a synthesis of a nucleic acid having a desirable sequence is completed, an amine compound is reacted to deprotect a protecting group of a phosphorus part. Examples of the amine compound include, for example, diethylamine and the others as described in JP 4705716 B2.

The protecting group of 5' hydroxyl group of a nucleoside incorporated in the last stage of an elongation may be used for a column purification with 5' protecting group as a tag after the below-mentioned procedures of a cutting out from a solid support and a deprotection of a protecting group, or alternatively, the protecting group of 5' hydroxyl group may be deprotected after the column purification.

Further, using aqueous ammonia or amine compound or the others, for example, an oligo nucleotide chain is recovered by cutting it out from a solid support as shown in the above Scheme 2. Examples of the amine compound include methylamine, ethylamine, propylamine, isopropylamine, ethylenediamine, diethylamine, and the others.

Examples of the nucleic acid oligomer which can be prepared according to the process of the present invention include those wherein a nucleoside contained in the nucleic acid oligomer is a RNA, a DNA, a RNA of 2'-O-MOE, 2'-O-Me, or 2'-F, and a LNA, which is not limited thereto.

For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included.

As typical examples of nucleic acid oligomer which can be used in the process of the present invention, the following examples are indicated in addition to examples described in working examples, which are not limited thereto.

Hereinafter, in a description of a sequence, U is uridine, C is cytidine, A is adenosine, or G is guanosine.

A nucleic acid oligomer having the following sequences (B) and (C) as described in WO 2019/060442 is exemplified.

Sequence (B) : 5'-AUGGAAUmACUCUUGGUUmACdTdT-3' (Antisense) (Sequence No. 3) 21 mer
Sequence (C): 5'-GUmAACmCmAAGAGUmAUmUmCmCmAUmdTdT-3' (Sense) (Sequence No. 4) 21 mer

In the sequence (B) and sequence (C), Um is 2'-O-metyluridine, Cm is 2'-O-methylcytidine, or dT is thymidine.

A nucleic acid oligomer as described in Daniel O'Reilly et al., Nucleic Acids Research, 2019, Vol. 47, No.2, 546-558 (refer to p. 553) is exemplified. Typical examples thereof include a nucleic acid oligomer having the following sequence (D).

Sequence (D): 5'-AGAGCCAGCCUUCUUAUUGUUUUAGAGCUAUGCUGU-3' (Sequence No. 5) 36 mer

A nucleic acid oligomer as described in JP 4965745 B2 is exemplified. Typical examples thereof include a nucleic acid oligomer having the following sequence (E).

Sequence (E): 5'-CCAUGAGAAGUAUGACAACAGCC-P-GGCUGUUGUCAUACUUCUCAUGGUU-3' 49 mer.

CCAUGAGAAGUAUGACAACAGCC (Sequence No. 6), GGCUGUUGUCAUACUUCUCAUGGUU (Sequence No. 7) .

In the Sequence (E), "P" is depicted by a partial structure separated by wavy lines in the following formula (A5) .

A nucleic acid oligomer having the following sequence (F) as described in Nucleic Acids Research, 2019, Vol. 47, No. 2: 547 is exemplified.

Sequence (F): 5'-ACAGCAUAGCAAGUUAAAAUAAGGCUAGUCCGUUAUCAACUUGAAAAAGUGGCACCGAGUCGGUGCU-3' (Sequence No. 8) 67 mer

A nucleic acid oligomer having the following sequence (G) as described in JP 2015-523856, 173 is exemplified.

Sequence (G): 5'-GUUUUCCCUUUUCAAAGAAAUCUCCUGGGCACCUAUCUUCUUAGGUGCCCUCCCUUGUUUAAACCUGACCAGUUAACCGGCUGGUUAGGUUUUU-3' (Sequence No. 9) 94 mer

A nucleic acid oligomer as described in JP 2017-537626 is exemplified. Typical examples thereof include a nucleic acid oligomer having the following sequences (F), (G), (H), and (J).

Sequence (F): 5'-AGUCCUCAUCUCCCUCAAGCGUUUUAGAGCUAGUAAUAGCAAGUUAAAAUAAGGCUAGUCCGUUAUCAACUUGAAAAAGUGGCACCGAGUCGGUGCUUUU-3' (Sequence No. 10) 100 mer

Sequence (G): 5'-GCAGAUGUAGUGUUUCCACAGUUUAAGAGCUAUGCUGGAAACAGCAUAGCAAGUUUAAAUAAGGCUAGUCCGUUAUCAACUUGAAAAAGUGGCACCGAGUCGGUGCUUUUUUU-3' (Sequence No. 11) 113 mer

Sequence (H): 5'-dAdGdTdCdCdTdCdAdTdCdTdCdCdCdTdCdAdAdGdCGUUUAAGAGCUAUGCUGGUAACAGCAUAGCAAGUUUAAAUAAGGCUAGUCCGUUAUCAACUUGAAAAAGUGGCACCGAGUCGGUGCUUUUUUU-3' (Sequence No. 12) 113 mer

In the sequence (H), dT is thymidine, dC is 2'-deoxycytidine, dA is 2'-deoxyadenosine, or dG is 2'-deoxyguanosine.

Sequence (J): 5'-AmsGmsUmsCCUCAUCUCCCUCAAGCGUUUAAGAGCUAUGCUGGUAACAGCAUAGCAAGUUUAAAUAAGGCUAGUCCGUUAUCAACUUGAAAAAGUGGCACCGAGUCGGUGCUUUUmsUmsUmsU-3' (Sequence No. 13) 113 mer

In the Sequence (J), Um is 2'-O-methyluridine, Am is 2'-O-methyladenosine, Gm is 2'-O-methylguanosine, or s is phosphorothioate modification.

EXAMPLES

Hereinafter, the resent invention is explained in more detail by working examples, and the present invention is not limited to these examples.

<Measurement method>

Various measurement methods used in the following tests are shown below.

(Measurement method 1: Measurement of oligonucleotide purity)

The measurement of purity of the oligonucleotide crude product after a solid phase synthesis was conducted by HPLC. The crude product was separated into each components by HPLC (wavelength 260 nm, column ACQUITY UPLC Oligonucleotide BEH C18, 2.1 mm × 100 mm, 1.7 um), and the purity of the oligonucleotide was calculated from the area value of major product as opposed to total area value of obtained chromatogram.

A HPLC measurement condition is shown in Table 1 below.

[Table 1]

| | |
|---|---|
| Column | ACQUITY UPLC Oligonucleotide BEH C18, 2.1 mm × 100 mm, 1.7 μm |
| Flow rate | 0.2 mL/min |
| Detection wavelength | 260 nm |
| Mobile phase A | 100 mM aqueous hexylamine acetate (pH = 7.0) |

(continued)

| | |
|---|---|
| Mobile phase B | 100 mM aqueous hexylamine acetate : acetonitrile = 1:4(v) |
| Gradient condition | B conc. 43%(0 min) -56%(70 min) -90%(70.01 min) -90%(75 min) -43%(75.01 min) -43% (90 min) |
| Column temperature | 80°C |

(Measurement method 2: Measurement of oligonucleotide yield)

$OD_{260}$ of the above crude product was measured. $OD_{260}$ represents an absorbance at UV260 nm per 10 mm optical path length in a 1 mL solution (pH = 7.5). Since it is generally known that 1 OD = 40 μg for RNA, the yield was calculated based on the above measured value of $OD_{260}$. Further, the yield of the products per unit volume of solid support was calculated. For the Examples 1 to 5 and Comparative Examples 1 and 2, each relative yield as opposed to the yield of Example 1 was calculated.

(Measurement method 3: Measurement of oxygen concentration)

The oxygen concentration of atmosphere (air phase) in the reaction system was measured by PACK KEEPER (Residual Oxygen Meter) (manufactured by IIJIMA ELECTRONICS CORP.). Before the measurement of oxygen concentration, the device was calibrated by measurement of oxygen concentration in air or pure nitrogen, and a needle attached to the device was then inserted into a container such as a flask covered with a septum or the like, and the oxygen concentration of air phase in reaction system was measured. The measured value of the oxygen concentration was displayed in real time, and the oxygen concentration at the time point at which the measured value was stable was made the oxygen concentration of the atmosphere.

(Measurement method 4: Measurement method of enzymatic decomposition product of oligonucleotide)

The measurement of an enzymatic decomposition product of a crude oligonucleotide product after solid-phase synthesis was carried out by HPLC. The decomposition product was separated into each component by HPLC (wavelength 260 nm, column Develisil, ODS-UG-5, 4.6 × 250 mm, 5 um), and the HPLC area normalization value of adenosine represented by formula (A15) and adenosine cyanoethyl adducts represented by (A16) respectively as opposed to the total area values of the obtained chromatogram was calculated. The term of "HPLC area normalization value" as described herein refer to as "HPLC area percentage value".

A HPLC measurement condition is shown in Table 2 below.

[Table 2]

| | |
|---|---|
| Column | Develisil oDS-UG-5, 4.6 mm × 250 mm, 5 μm |
| Flow rate | 1.0 mL/min |
| Detection wavelength | 260 nm |
| Mobile phase A | 10 mM aqueous ammonium acetate |
| Mobile phase B | Methanol : 10 mM aqueous ammonium acetate = 1:1 (v/v) |
| Gradient condition | B conc. 15%(0 min) -15%(10 min) -25%(20 min) - 25%(30 min)-40%(45 min)- 100% (65 min) - 100%(70 min) -15%(70.01 min) -15%(80 min) |
| Column temperature | 30°C |

Each structural formula of the adenosine or the cyanoethyl adducts of the adenosine as a component which is detected by HPLC due to an enzymatic decomposition of oligomer is represented by formula (A15) or formula (A16) respectively.

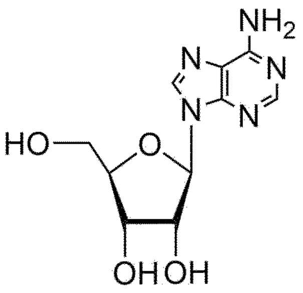

,

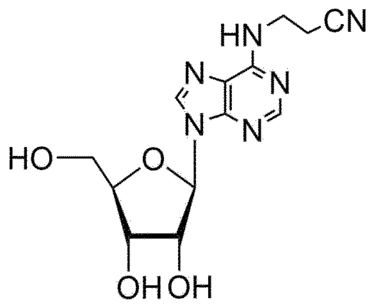

(A15) (A16)

(Enzymatic decomposition method)

The method for enzymatic decomposition of oligonucleotide is shown below.

An aqueous solution of the crude product of oligonucleotide which was adjusted to 0.5 mg/mL concentration 83 μL was placed in 2 mL vial, and an aqueous solution of 0.2 unit/pL Nuclease P (derived from Penicillium Citrinum) 2 μL was added thereto, and the mixture was incubated in a 60 °C incubator for 2 hours. Alkaline Phosphatase (derived from Calf Intestinal) 10 × Buffer 10 μL, and Alkaline Phosphatase (derived from Calf Intestinal) 5 μL were added thereto, and the mixture were incubated in a 56 °C incubator for 2 hours.

As a result of the enzymatic decomposition of oligonucleotide, the content of the adenosine cyanoethyl adduct as an impurity contained in oligonucleotide was calculated from HPLC normalization area value of formula (A15) and formula (A16) which was detected by HPLC analysis as described in the measurement method 4. The calculating method is show below.

$$[d] = [a] \times ([a] + [b]) \times 100 \times [c]$$

In the above equation, [a] represents a HPLC normalization area value of formula (A16) which is defined by HPLC analysis as described in the measurement method 4, [b] represents a HPLC normalization area value of formula (A15) which is defined by HPLC analysis as described in the measurement method 4, [c] represents the number of adenosine which is contained in the desired oligonucleotide, and [d] represents the content of adenosine cyanoethyl adduct as an impurity contained in oligomer, which is calculated from the HPLC normalization area value of formula (A15) and formula (A16) which are detected by HPLC analysis as described in the measurement method 4.

Solid phase synthesis of oligonucleotide

Sequence (I): 5'-AGCAGAGUACACACAGCAUAUACC-P-GGUAUAUGCUGUGUGUACUCUGCUUC-P-G-3' (Sequence Nos. 1 and 2) 53 mer

In the above sequence (I), "A" is represented by a partial structure separated by wavy lines in the following formula (A1). "C" is represented by a partial structure separated by wavy lines in the following formula (A2). "G" is represented by a partial structure separated by wavy lines in the following formula (A3). "U" is represented by a partial structure separated by wavy lines in the following formula (A4). "P" is represented by a partial structure separated by wavy lines in the following formula (A5). Here "A" at a 5' terminus is represented by a partial structure separated by wavy lines in the following formula (A6). Also "G" at a 3' terminus is represented by a partial structure separated by wavy lines in the following formula (A7) . Here the phosphoric acid in a structural formula may be its salt.

AGCAGAGUAC ACACAGCAUA UACC (Sequence No. 1)
GGUAUAUGCU GUGUGUACUC UGCUUC (Sequence No. 2)

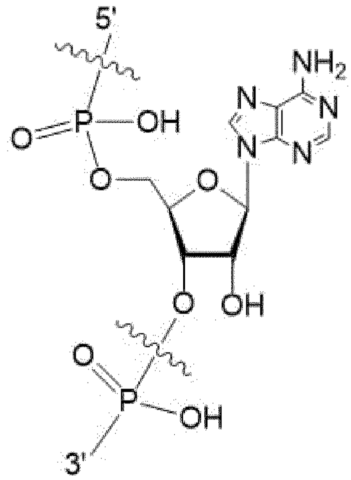

(A1)

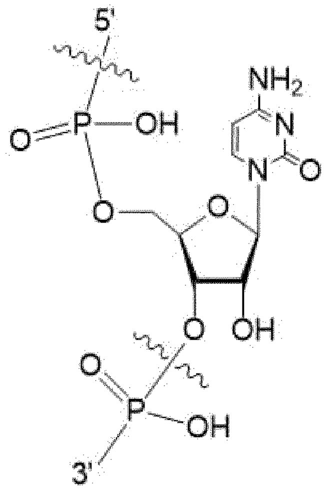

(A2)

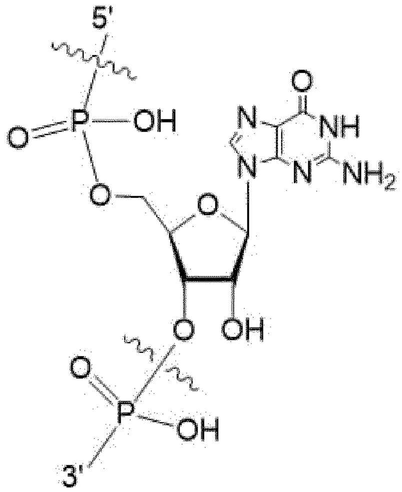

(A3)

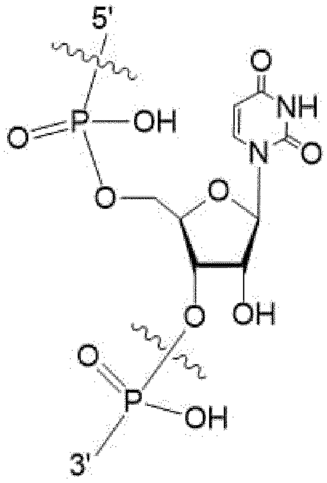

(A4)

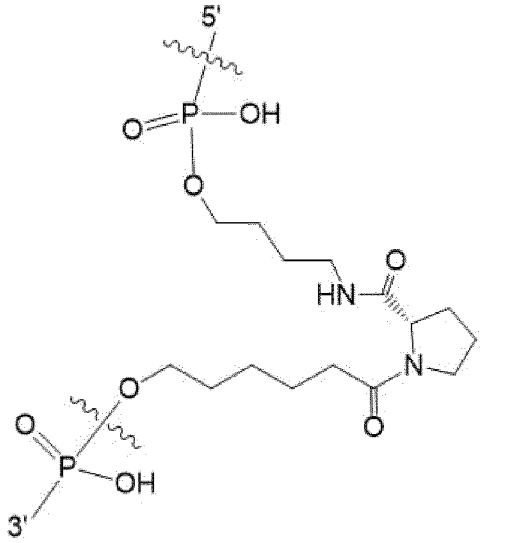

(A5)

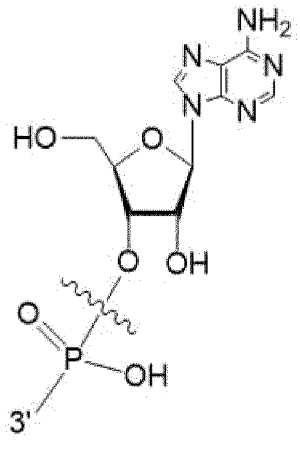

(A6)

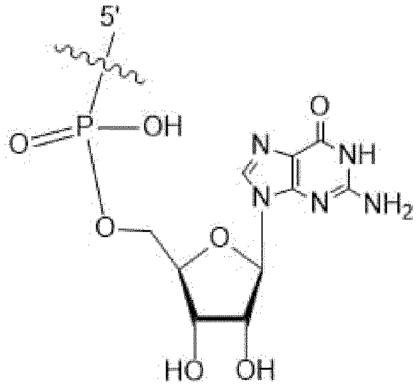

(A7)

Using a Controlled Pore Glass (CPG) as a solid support and an AKTA oligonucleotide plus 100 (manufactured by GE healthcare) as a nucleic acid synthesizer, according to a phosphoramidite solid phase synthesis method, an oligonucleotide composed by the above sequence (I) was synthesized from the 3' side to the 5' side. The synthesis was carried out on a scale of 77.89 μmol scale. Also in the synthesis, a uridine PMM amidite (Compound (A11)) (as described in the working Example 2 of US patent publication 2012/0035246), a cytidine EMM amidite (Compound (A9)) (as described in the working Example 3), an adenosine EMM amidite (Compound (A8)) (as described in the working Example 4), a guanosine EMM amidite (Compound (A10)) (as described in the working Example 5), Compound (A12) (as described in WO 2017/188042), $N^6$-acetyl-5'-O-(4,4'-dimethoxytrityl)-2'-O-(2-cyanoethoxymethyl)adenosine 3'-O-(2-cyanoethyl N,N-diisopropyl phosphoramidite (Compound (A15) (as described in the working Exmaple 9 of JP 5157168 B), $N^2$-acetyl-5'-O-(4,4'-dimethoxytrityl)-2'-O-(2-cyanoethoxymethyl)adenosine 3'-O-(2-cyanoethyl N,N-diisopropyl phosphoramidite (Compound (A17) (as described in the working Example 8 thereof), $N^4$-acetyl-5'-O-(4,4'-dimethoxytrityl)-2'-O-(2-cyanoethoxymethyl)cytidine 3'-O-(2-cyanoethyl N,N-diisopropyl phosphoramidite (Compound (A16) (as described in the working Example 5 thereof), and 5'-O-(4,4'-dimethoxytrityl)-2'-O-(2-cyanoethoxymethyl)uridine 3'-O-(2-cyanoethyl N,N-diisopropyl phosphoramidite (A18) (as described in the working Example 2 thereof) were used, and a high-purity solution of trichloro acetic acid in toluene was used as a deblocking solution, and 5-benzylmercapto-1H-tetrazole was used as a condensation agent, and iodine solution was used as an oxidizing agent, and a phenoxy acetic anhydride solution and a N-methyl imidazole solution were used as a capping solution. After a completion of a nucleic acid elongation, a diethylamine solution was acted to a nucleic acid on a support so as to deprotect selectively a cyanoethyl protecting group in a phosphoric acid part.

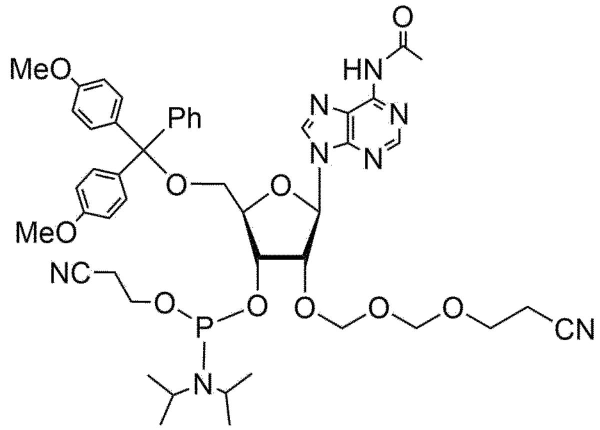

(A8)

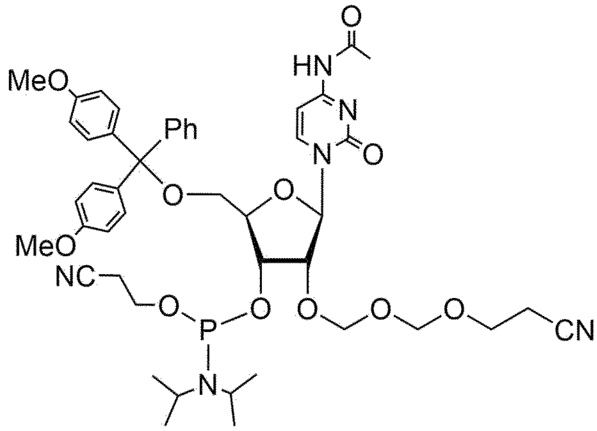

(A9)

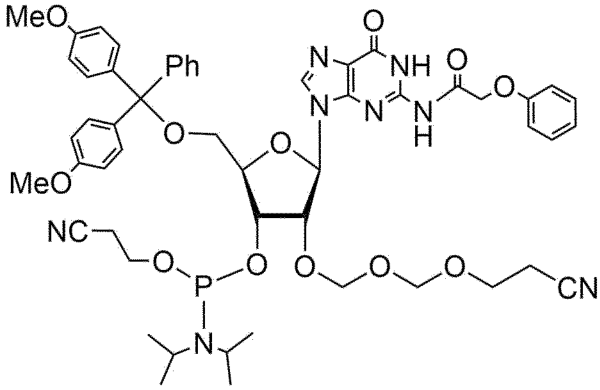

(A10)

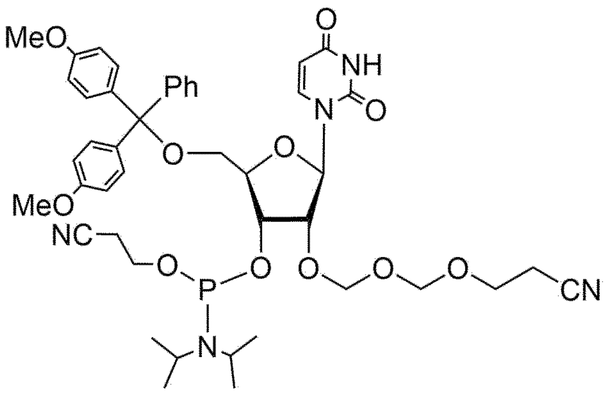

(A11)

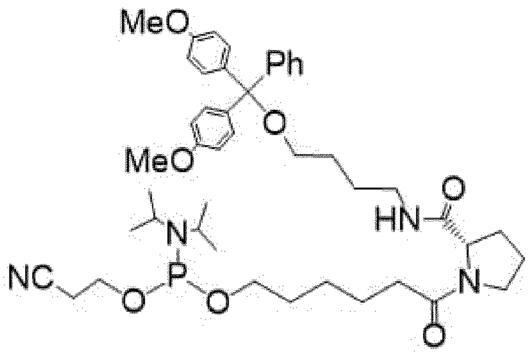

(A12)

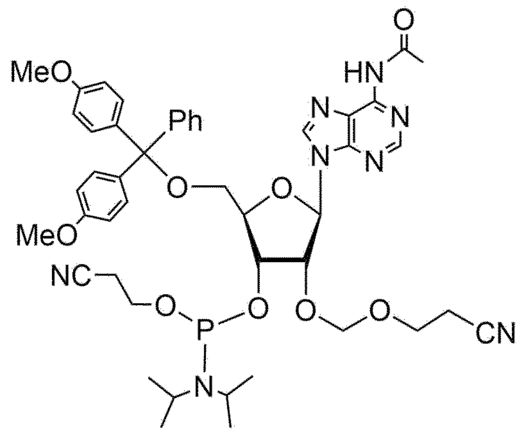

(A15)

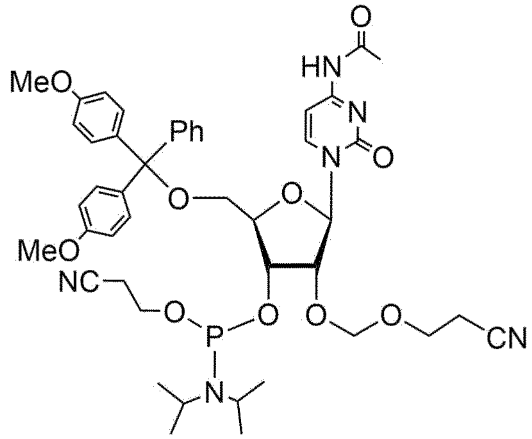

(A16)

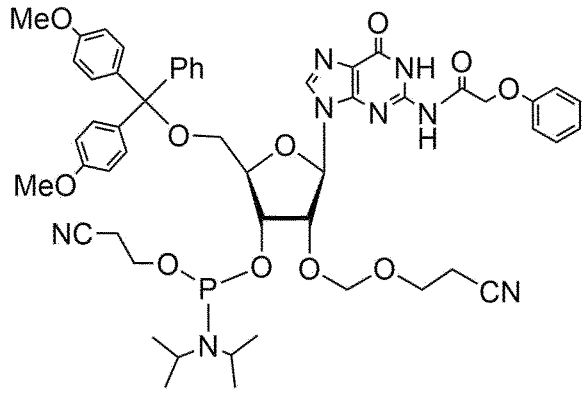

(A17)

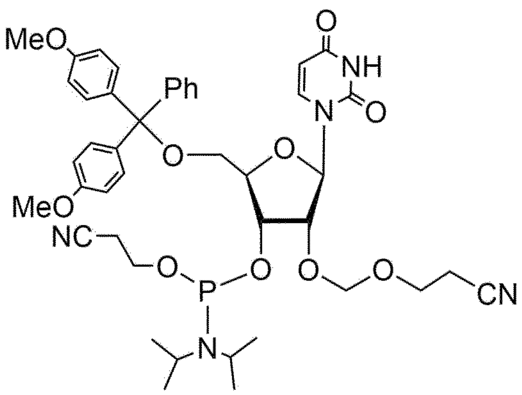

(A18)

Next, specific preparation examples of oligonucleotide (nucleic acid oligomer) prepared according to the process of the present invention are shown. Here, in the following examples, the oligonucleotides prepared according to the process of the present invention are oligonucleotide having sequence (I) shown by sequence Nos. 1 and 2.

Also, the guanosine derivatives as described in the following Examples and Comparative Examples represent the compounds represented by the following structural formula. A circle as depicted in the following structural formula represents CPG schematically.

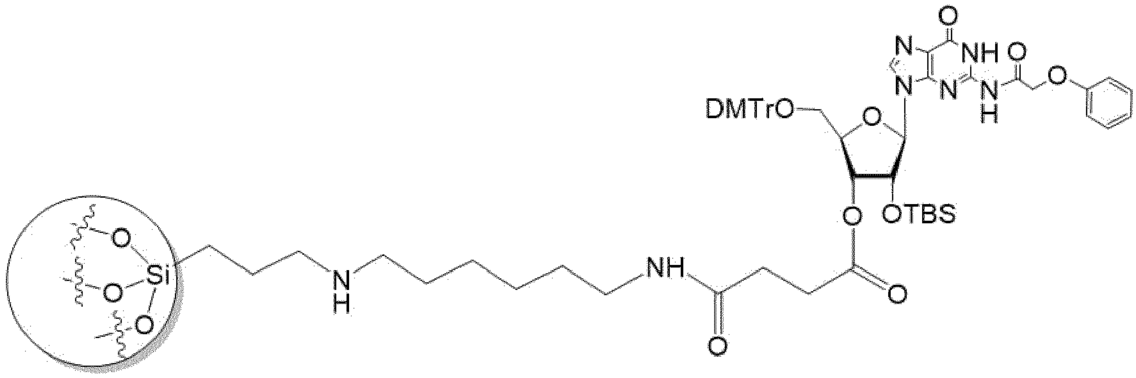

(Example 1)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.53 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further, 1M tetra-n-butylammonium fluoride (TBAF) in dimethyl sulfoxide solution 1.13 g (which was dehydrated with molecular sheave 4A) (the amount of TBAF was 12.7 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-EMM protecting group. The crude product was obtained by a precipitation procedure. The yield was 13.0 mg, and the purity was 58 %. As for the resulting crude product, the purity of the oligonucleotide was measured according to the method described in the above measurement method 1, and the yield of the oligonucleotide was measured according to the method described in the above measurement method 2.

(Example 2)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 0.98 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in

the system was substituted with argon gas by flowing argon gas, and an oxygen concentration in gas phase was made 5%. Here the oxygen concentration in the gas phase was measured according to the method described in the above measurement method 3. Further, 1M tetra-n-butylammonium fluoride (TBAF) in dimethyl sulfoxide solution 0.76 g (which was dehydrated with molecular sheave 4A) (the amount of TBAF was 13.2 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-EMM protecting group. The crude product was obtained by a precipitation procedure. The yield was 8.3 mg, and the purity was 54 %. As for the resulting crude product, the purity of the oligonucleotide was measured according to the method described in the above measurement method 1, and the yield of the oligonucleotide was measured according to the method described in the above measurement method 2.

(Example 3)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.00 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 10%. Here the oxygen concentration in the gas phase was measured according to the method described in the above measurement method 3. Further, 1M tetra-n-butylammonium fluoride (TBAF) in dimethyl sulfoxide solution 0.74 g (which was dehydrated with molecular sheave 4A) (the amount of TBAF was 12.7 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-EMM protecting group. The crude product was obtained by a precipitation procedure. The yield was 8.4 mg, and the purity was 49 %. As for the resulting crude product, the purity of the oligonucleotide was measured according to the method described in the above measurement method 1, and the yield of the oligonucleotide was measured according to the method described in the above measurement method 2.

(Example 4)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.00 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 15%. Here the oxygen concentration in the gas phase was measured according to the method described in the above measurement method 3. Further, 1M tetra-n-butylammonium fluoride (TBAF) in dimethyl sulfoxide solution 0.75 g (which was dehydrated with molecular sheave 4A) (the amount of TBAF was 12.9 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-EMM protecting group. The crude product was obtained by a precipitation procedure. The yield was 8.4 mg, and the purity was 46 %. As for the resulting crude product, the purity of the oligonucleotide was measured according to the method described in the above measurement method 1, and the yield of the oligonucleotide was measured according to the method described in the above measurement method 2.

(Reference Example 1)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.51 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for measurement equipped with Oxygen Meter were pierced onto the septum, and an oxygen concentration in gas phase was made 21%. Here the oxygen concentration in the gas phase was measured according to the method described in the above measurement method 3. Further, 1M tetra-n-butylammonium fluoride (TBAF) in dimethyl sulfoxide solution 1.09 g (which was dehydrated with molecular sheave 4A) (the amount of TBAF was 12.4 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-EMM protecting group. The crude product was obtained by a precipitation procedure. The yield was 13.0 mg, and the purity was 45 %. As for the resulting crude product, the purity of the oligonucleotide was measured according to the method described in the above measurement method 1, and the yield of the oligonucleotide was measured according to the method described in the above measurement method 2.

(Example 5)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.53 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further, 1M tetra-n-butylammonium fluoride (TBAF) in dimethyl sulfoxide solution 1.13 g (which was dehydrated with molecular sheave 4A) (the amount of TBAF was 12.7 moles per 1 mole of a protecting group) was flown using a syringe onto a surface of oligonucleotide solution at 33°C under stirring with a stirrer within 1 minute using a syringe, and the resulting mixture was kept its temperature for 4 hours at 33°C to deprotect a 2'-cyanoethoxymethoxy (CEM) protecting group. The crude product was obtained by a precipitation procedure. The yield was 13.5 mg, and the purity was 48 %. As for the resulting crude product, the purity of the oligonucleotide was measured according to the method described in the above measurement method 1, and the yield of the oligonucleotide was measured according to the method described in the above measurement method 2.

(Example 6)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.53 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of

29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 5%. Here the oxygen concentration in the gas phase was measured according to the method described in the above measurement method 3. Further, 1M tetra-n-butylammonium fluoride (TBAF) in dimethyl sulfoxide solution 1.14 g (which was dehydrated with molecular sheave 4A) (the amount of TBAF was 12.7 moles per 1 mole of a protecting group) was flown onto a surface of oligonucleotide solution at 33°C under stirring with a stirrer within 1 minute using a syringe, and the resulting mixture was kept its temperature for 4 hours at 33°C to deprotect a 2'-cyanoethoxymethoxy (CEM) protecting group. The crude product was obtained by a precipitation procedure. The yield was 13.5 mg, and the purity was 46 %. As for the resulting crude product, the purity of the oligonucleotide was measured according to the method described in the above measurement method 1, and the yield of the oligonucleotide was measured according to the method described in the above measurement method 2.

(Example 7)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.50 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 10%. Here the oxygen concentration in the gas phase was measured according to the method described in the above measurement method 3. Further, 1M tetra-n-butylammonium fluoride (TBAF) in dimethyl sulfoxide solution 1.14 g (which was dehydrated with molecular sheave 4A) (the amount of TBAF was 13.0 moles per 1 mole of a protecting group) was flown onto a surface of oligonucleotide solution at 33°C under stirring with a stirrer within 1 minute using a syringe, and the resulting mixture was kept its temperature for 4 hours at 33°C to deprotect a 2'-cyanoethoxymethoxy (CEM) protecting group. The crude product was obtained by a precipitation procedure. The yield was 13.4 mg, and the purity was 45 %. As for the resulting crude product, the purity of the oligonucleotide was measured according to the method described in the above measurement method 1, and the yield of the oligonucleotide was measured according to the method described in the above measurement method 2.

(Example 8)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.50 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 15%. Here the oxygen concentration in the gas phase was measured according to the method described in the above measurement method 3. Further, 1M tetra-n-butylammonium fluoride (TBAF) in dimethyl sulfoxide solution 1.11 g (which was dehydrated with molecular sheave 4A) (the amount of TBAF was 12.7 moles per 1 mole of a protecting group) was flown onto a surface of oligonucleotide solution at 33°C under stirring with a stirrer within 1 minute using a syringe, and the resulting mixture was kept its temperature for 4 hours at 33°C to deprotect a 2'-cyanoethoxymethoxy (CEM) protecting group. The crude product was obtained by a precipitation procedure. The yield was 13.3 mg, and the purity was 44 %. As for the resulting crude product, the purity of the oligonucleotide was measured according to the

method described in the above measurement method 1, and the yield of the oligonucleotide was measured according to the method described in the above measurement method 2.

(Reference Example 2)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.51 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for measurement equipped with Oxygen Meter were pierced onto the septum, and an oxygen concentration in gas phase was made 21%. Here the oxygen concentration in the gas phase was measured according to the method described in the above measurement method 3. Further, 1M tetra-n-butylammonium fluoride (TBAF) in dimethyl sulfoxide solution 1.10 g (which was dehydrated with molecular sheave 4A) (the amount of TBAF was 12.5 moles per 1 mole of a protecting group) was added dropwise to a surface of oligonucleotide solution at 33°C under stirring with a stirrer over 1 hour using a syringe pump (manufactured by KDScientific Inc.), and the resulting mixture was kept its temperature for 4 hours at 33°C to deprotect a 2'-cyanoethoxymethoxy (CEM) protecting group. The crude product was obtained by a precipitation procedure. The yield was 13.3 mg, and the purity was 43 %. As for the resulting crude product, the purity of the oligonucleotide was measured according to the method described in the above measurement method 1, and the yield of the oligonucleotide was measured according to the method described in the above measurement method 2.

(Reference Example 3)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.51 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for measurement equipped with Oxygen Meter were pierced onto the septum, and an oxygen concentration in gas phase was made 21%. Here the oxygen concentration in the gas phase was measured according to the method described in the above measurement method 3. Further, 1M tetra-n-butylammonium fluoride (TBAF) in dimethyl sulfoxide solution 1.10 g (which was dehydrated with molecular sheave 4A) (the amount of TBAF was 12.5 moles per 1 mole of a protecting group) was flown to a surface of oligonucleotide solution at 33°C under stirring with a stirrer within 1 minute using a syringe, and the resulting mixture was kept its temperature for 4 hours at 33°C to deprotect a 2'-cyanoethoxymethoxy (CEM) protecting group. The crude product was obtained by a precipitation procedure. The yield was 13.3 mg, and the purity was 43 %. As for the resulting crude product, the purity of the oligonucleotide was measured according to the method described in the above measurement method 1, and the yield of the oligonucleotide was measured according to the method described in the above measurement method 2.

(Example 9)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 6.04 μmol parts of the resulting solution was collected in an egg-plant shaped flask having a volume of 100 mL and a caliber of 29 mm, and a stir bar having a diameter of 15

mm and acetonitrile 0.26 g were further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 0°C under ice for 20 minutes, a mixed solution of 1M tetra-n-butylammonium fluoride (TBAF) in dimethyl sulfoxide solution 4.49 g (which was dehydrated with molecular sheave 4A) (the amount of TBAF was 12.8 moles per 1 mole of a protecting group) and acetonitrile 0.90 g was added dropwise into a surface of oligonucleotide solution at 0°C under stirring with a stirrer over 1 hour using a syringe pump (manufactured by KDScientific Inc.), and after the addition dropwise, the temperature of the resulting mixture was raised to 43°C, and the resulting mixture was kept its temperature for 4 hours to deprotect a 2'-EMM protecting group. The crude product was obtained by a precipitation procedure. The yield was 52.4 mg, and the purity was 58 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1945 % or 0.0064 % respectively. Here since the number of the adenosine in the desirable oligonucleotide was thirteen (13), the contents of the impurity materials were calculated using the above-mentioned equation, and are shown in Table 4.

(Example 10)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 0.99 μmol parts of the resulting solution was collected in an egg-plant shaped flask having a volume of 50 mL and a caliber of 29 mm, and a stir bar having a diameter of 15 mm and acetonitrile 0.12 g were further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 0°C under ice for 20 minutes, a mixed solution of 1M tetra-n-butylammonium fluoride (TBAF) in dimethyl sulfoxide solution 0.81 g (which was dehydrated with molecular sheave 4A and cooled under ice) (the amount of TBAF was 14.0 moles per 1 mole of a protecting group) and acetonitrile 0.20 g was flown onto a surface of oligonucleotide solution at 0°C under stirring with a stirrer within 1 minute using a syringe, and after keeping the temperature of the mixture at 0°C for 1 hour under stirring, the temperature of the resulting mixture was raised to 33°C, and the resulting mixture was kept its temperature for 4 hours to deprotect a 2'-EMM protecting group. The crude product was obtained by a precipitation procedure. The yield was 8.5 mg, and the purity was 58 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1150 % or 0.0052 % respectively. Here since the number of the adenosine in the desirable oligonucleotide was thirteen (13), the contents of the impurity materials were calculated using the above-mentioned equation, and are shown in Table 4.

(Example 11)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g

and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 0.99 μmol parts of the resulting solution was collected in an egg-plant shaped flask having a volume of 50 mL and a caliber of 29 mm, and a stir bar having a diameter of 15 mm and acetonitrile 0.12 g were further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 10°C for 20 minutes, and a mixed solution of 1M tetra-n-butylammonium fluoride (TBAF) in dimethyl sulfoxide solution 0.77 g (which was dehydrated with molecular sheave 4A and cooled to 10°C) (the amount of TBAF was 13.2 moles per 1 mole of a protecting group) and acetonitrile 0.18 g was flown onto a surface of oligonucleotide solution at 10°C under stirring with a stirrer within 1 minute using a syringe, and after keeping the temperature of the mixture at 10°C for 1 hour under stirring, the temperature of the resulting mixture was raised to 33°C, and the resulting mixture was kept its temperature for 4 hours to deprotect a 2'-EMM protecting group. The crude product was obtained by a precipitation procedure. The yield was 8.5 mg, and the purity was 58 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1972 % or 0.0056 % respectively. Here since the number of the adenosine in the desirable oligonucleotide was thirteen (13), the contents of the impurity materials were calculated using the above-mentioned equation, and are shown in Table 4.

(Example 12)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.00 μmol parts of the resulting solution was collected in an egg-plant shaped flask having a volume of 50 mL and a caliber of 29 mm, and a stir bar having a diameter of 15 mm and acetonitrile 0.12 g were further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 20°C for 20 minutes, and a mixed solution of 1M tetra-n-butylammonium fluoride (TBAF) in dimethyl sulfoxide solution 0.82 g (which was dehydrated with molecular sheave 4A and wormed to 20°C) (the amount of TBAF was 14.0 moles per 1 mole of a protecting group) was flown onto a surface of oligonucleotide solution at 20°C under stirring with a stirrer within 1 minute using a syringe, and after keeping the temperature of the mixture at 20°C for 1 hour under stirring, the temperature of the resulting mixture was raised to 33°C, and the resulting mixture was kept its temperature for 4 hours to deprotect a 2'-EMM protecting group. The crude product was obtained by a precipitation procedure. The yield was 8.2 mg, and the purity was 57 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.3364 % or 0.0246 % respectively. Here since the number of the adenosine in the desirable oligonucleotide was thirteen (13), the contents of the impurity materials were calculated using the above-mentioned equation, and are shown in Table 4.

(Example 13)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.03 μmol parts of the resulting solution was collected in an egg-plant shaped flask having a volume of 50 mL and a caliber of 29 mm, and a stir bar having a diameter of 15 mm and acetonitrile 0.12 g were further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 25°C for 20 minutes, 1M tetra-n-butylammonium fluoride (TBAF) in dimethyl sulfoxide solution 0.77 g (which was dehydrated with molecular sheave 4A and wormed to 25°C) (the amount of TBAF was 12.8 moles per 1 mole of a protecting group) was flown onto a surface of oligonucleotide solution at 25°C under stirring with a stirrer within 1 minute using a syringe, and after keeping the temperature of the mixture at 25°C for 1 hour under stirring, the temperature of the resulting mixture was raised to 33°C, and the resulting mixture was kept its temperature for 4 hours to deprotect a 2'-EMM protecting group. The crude product was obtained by a precipitation procedure. The yield was 8.4 mg, and the purity was 56 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1564 % or 0.0351 % respectively. Here since the number of the adenosine in the desirable oligonucleotide was thirteen (13), the contents of the impurity materials were calculated using the above-mentioned equation, and are shown in Table 4.

(Example 14)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.53 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further, 1M tetra-n-butylammonium fluoride (TBAF) in dimethyl sulfoxide solution 1.13 g (which was dehydrated with molecular sheave 4A) (the amount of TBAF was 12.7 moles per 1 mole of a protecting group) was added dropwise into a surface of oligonucleotide solution at 33°C under stirring with a stirrer over 1 hour using a syringe pump (manufactured by KDScientific Inc.), and the resulting mixture was kept at 33°C for 4 hours to deprotect a 2'-EMM protecting group. The crude product was obtained by a precipitation procedure. The yield was 13.0 mg, and the purity was 58 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1401 % or 0.0245 % respectively. Here since the number of the adenosine in the desirable oligonucleotide was thirteen (13), the contents of the impurity materials were calculated using the above-mentioned equation, and are shown in Table 4.

(Reference Example 4)

Using CPG on which 77.89 μmol guanosine derivative was supported, and each amidite represented by formula (A8), formula (A9), formula (A10), formula (A11), or formula (A12) respectively, a solid-phase synthesis of sequence (I) was carried out by a AKTA oligopilot plus 100. Thereafter, a CPG support on which 30.06 μmol of oligonucleotide was supported was collected, and the oligonucleotide was liberated from the solid support using aqueous ammonia 10.17 g and ethanol 3.03 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.47 g, and 1.55 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further the solution was stirred at 33°C, and 1M tetra-n-butylammonium fluoride (TBAF) in dimethyl sulfoxide solution 1.14 g (which was dehydrated with molecular sheave 4A) (the amount of TBAF was 12.7 moles per 1 mole of a protecting group) was flown onto a surface of oligonucleotide solution at 25°C under stirring with a stirrer within 1 minute using a syringe, and the resulting mixture was kept at 33°C for 4 hours to deprotect a 2'-EMM protecting group. The crude product was obtained by a precipitation procedure. The yield was 13.2 mg, and the purity was 54 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.1927 % or 0.1252 % respectively. Here since the number of the adenosine in the desirable oligonucleotide was thirteen (13), the contents of the impurity materials were calculated using the above-mentioned equation, and are shown in Table 4.

(Example 15)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 5.94 μmol parts of the resulting solution was collected in an egg-plant shaped flask having a volume of 100 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further, 1M tetra-n-butylammonium fluoride (TBAF) in dimethyl sulfoxide solution 4.48 g (which was dehydrated with molecular sheave 4A) (the amount of TBAF was 13.0 moles per 1 mole of a protecting group) was flown onto a surface of oligonucleotide solution at 25°C under stirring with a stirrer within 1 minute using a syringe, and after keeping the temperature of the mixture at 25°C for 1 hour under stirring, the temperature of the resulting mixture was raised to 33°C, and the resulting mixture was kept its temperature for 4 hours to deprotect a 2'-cyanoethoxymethoxy (CEM) protecting group. The crude product was obtained by a precipitation procedure. The yield was 52.0 mg, and the purity was 50 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.4413 % or 0.0253 % respectively. Here since the number of the adenosine in the desirable oligonucleotide was thirteen (13), the contents of the impurity materials were calculated using the above-mentioned equation, and are shown in Table 4.

(Reference Example 5)

Using CPG on which 77.89 μmol guanosine derivative was supported, and each amidite represented by formula (A15), formula (A16), formula (A17), formula (A18), or formula (A12) respectively, a solid-phase synthesis of sequence (I) was carried out by a AKTA oligopilot plus 100. Thereafter, a CPG support on which 30.04 μmol of oligonucleotide was supported was collected, and the oligonucleotide was liberated from the solid support using aqueous ammonia 10.17 g and ethanol 3.06 g, and the support was filtered off, and the filtrates containing the liberated oligonucleotide were concentrated to dryness. Next, the liberated oligonucleotide was solubilized in 13.21 g of dimethyl sulfoxide, and thereto were added nitromethane 0.22 g and acetonitrile 2.42 g, and 1.53 μmol parts of the resulting solution was collected in an egg-plant shaped flask having a volume of 50 mL and a caliber of 29 mm, and a stir bar having a diameter of 15 mm was further placed therein, and a flask was then sealed tightly by covering with a septum having a caliber of 29 mm. Further, a needle for blowing argon gas from an argon cylinder into the system, a needle for removing blown argon, and further a needle for measurement equipped with Oxygen Meter were pierced onto the septum, and the atmosphere in the system was substituted with argon gas by flowing argon gas, and an oxygen concentration in gas phase was made 0%. Here the oxygen concentration in the gas phase was measured according to the method described in the above measurement method 3. Further 1M tetra-n-butylammonium fluoride (TBAF) in dimethyl sulfoxide solution 1.13 g (which was dehydrated with molecular sheave 4A) (the amount of TBAF was 12.7 moles per 1 mole of a protecting group) was flown onto a surface of oligonucleotide solution at 33°C under stirring with a stirrer within 1 minute using a syringe, and the resulting mixture was kept at 33°C for 4 hours to deprotect a 2'-cyanoethoxymethoxy (CEM) protecting group. The crude product was obtained by a precipitation procedure. The yield was 13.5 mg, and the purity was 48 %. As for the resulting crude product, the purity of the oligonucleotide was measured according to the method described in the above measurement method 1, and the yield of the oligonucleotide was measured according to the method described in the above measurement method 2. Further the oligonucleotide was subjected to a decomposition reaction according to the method described in the above-mentioned enzymatic decomposition, and the HPLC area normalization value of formula (A15) and (A16) respectively was calculated using the method described in the above-mentioned measurement method 4 to obtain 31.3067 % or 0.0395 % respectively. Here since the number of the adenosine in the desirable oligonucleotide was thirteen (13), the contents of the impurity materials were calculated using the above-mentioned equation, and are shown in Table 4.

The measurement results are shown in Table 3 below.

[Table 3]

| | Protecting group at 2' position | Oxygen concentration | Duration required for TBAF addition | Duration of keeping temperature after completion of TBAF addition | Reaction Temperature | Relative yield per unit volume | Purity of nucleic acid by HPLC measurement |
|---|---|---|---|---|---|---|---|
| Ex.1 | EMM | 0% | 1 hr. | 4 hrs. | 33°C | 0.96 | 58% |
| Ex.2 | EMM | 5% | 1 hr. | 4 hrs. | 33°C | 0.95 | 54% |
| Ex. 3 | EMM | 10% | 1 hr. | 4 hrs. | 33°C | 0.95 | 49% |
| Ex.4 | EMM | 15% | 1 hr. | 4 hrs. | 33°C | 0.96 | 46% |
| Ref. Ex.1 | EMM | 21% | 1 hr. | 4 hrs. | 33°C | 0.97 | 45% |
| | | | | | | | |
| Ex. 5 | CEM | 0% | within 1 min. | 4 hrs. | 33°C | 1.00 | 48% |
| Ex. 6 | CEM | 5% | within 1 min. | 4 hrs. | 33°C | 1.00 | 46% |
| Ex. 7 | CEM | 10% | within 1 min. | 4 hrs. | 33°C | 1.01 | 45% |
| Ex. 8 | CEM | 15% | within 1 min. | 4 hrs. | 33°C | 1.00 | 44% |

(continued)

| | Protecting group at 2' position | Oxygen concentration | Duration required for TBAF addition | Duration of keeping temperature after completion of TBAF addition | Reaction Temperature | Relative yield per unit volume | Purity of nucleic acid by HPLC measurement |
|---|---|---|---|---|---|---|---|
| Ref. Ex.2 | CEM | 21% | 1 hr. | 4 hrs. | 33°C | 1.00 | 43% |
| Ref. Ex. 3 | CEM | 21% | within 1 min. | 4 hrs. | 33°C | 1.00 | 43% |

[Table 4]

| | Protecting group at 2' position | Oxygen concentration | Temperature at TBAF addition | Duration required for TBAF addition | Duration of keeping temperature after completion of TBAF addition | Relative yield per unit volume | Purity of nucleic acid by HPLC measurement | Impurity content of adenosine cyanoethyl adducts in nucleic acid |
|---|---|---|---|---|---|---|---|---|
| Ex. 9 | EMM | 0% | 0°C | 1 hr. | 4 hrs. | 0.98 | 58% | 0.27 % |
| Ex. 10 | EMM | 0% | 0°C | within 1 min. | 4 hrs. after 1 hr. | 0.97 | 58% | 0.22 % |
| Ex. 11 | EMM | 0% | 10°C | within 1 min. | 4 hrs. after 1 hr. | 0.97 | 58% | 0.23 % |
| Ex. 12 | EMM | 0% | 20°C | within 1 min. | 4 hrs. after 1 hr. | 0.93 | 57% | 1.02 % |
| Ex. 13 | EMM | 0% | 25°C | within 1 min. | 4 hrs. after 1 hr. | 0.92 | 56% | 1.46 % |
| Ex. 14 | EMM | 0% | 33°C | 1 hr. | 4 hrs. | 0.96 | 58% | 1.02 % |
| Ref. Ex.4 | EMM | 0% | 33°C | within 1 min. | 4 hrs. | 0.97 | 54% | 5.20 % |
| | | | | | | | | |
| Ex. 15 | CEM | 0% | 25°C | within 1 min. | 4 hrs. after 1 hr. | 0.99 | 50% | 1.05 % |
| Ref. Ex.5 | CEM | 0% | 33°C | within 1 min. | 4 hrs. | 1.00 | 48% | 1.64 % |

As shown in the above Table 2, it is confirmed that when the deprotection reaction of a protecting group of hydroxy group of a ribose which is contained in oligonucleotide as described in Description was carried out under an atmosphere of inert gases containing 15 % or less of oxygen concentration, compared to the case where the reaction was carried out under the atmosphere containing more than 15 % of oxygen concentration, the deprotection reaction proceeded more effectively, and further, the deprotection reaction proceeded further more effectively by contacting the reaction solution with TBAF at 25 °C or less as the temperature of the reaction system as compared to the case where the reaction solution is contacted with TBAF at more 25 °C as the temperature of the reaction system, and the deprotection reaction proceeded further more effectively by increasing to 30 minutes or more as the duration required for the TBAF addition as compared to the case where the duration required for the TBAF addition is made 1 minute or less, and as a result, the purity of the resulting deprotected oligonucleotide was high.

Industrial Applicability

The present invention provides an efficient process for preparing a nucleic acid oligomer. The improved purity of the nucleic acid oligomer which is prepared according to the process of nucleic acid oligomer of the present invention can be expected.

[Free text of Sequence Listing]

Sequence Nos. 1 to 13 in Sequence Listing represent a nucleotide sequence of oligonucleotides that are prepared according to the process of the present invention.

[Sequence Listing]

**Claims**

1. A process for preparing a nucleic acid oligomer represented by formula (4):

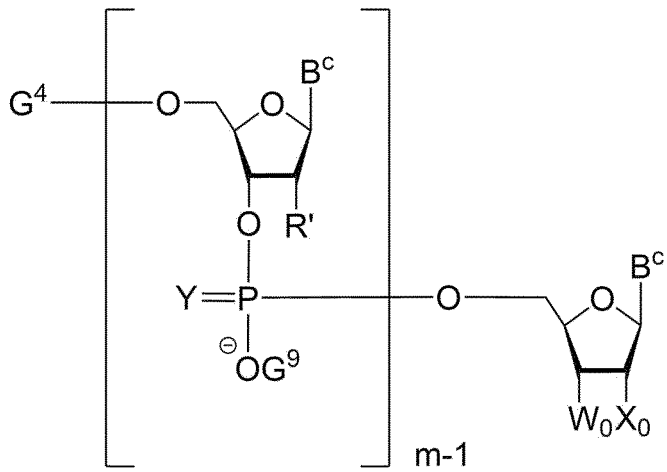

(4)

[wherein

R' is identical to or different from each other and each independently represents a hydroxy group, a hydrogen atom, a fluorine atom, a methoxy group, a 2-methoxyethyl group, or OQ' group,
Q' is identical to or different from each other and each independently represents a methylene group which is attached to a carbon atom at 4' position of ribose, an ethylene group which is attached to a carbon atom at 4' position of ribose, or an ethylidene group which is attached to a carbon atom at 4' position of ribose,
the definitions of substituents $G^4$, $G^5$, Y, $B^c$ and m of the formula (4) are the same as those defined in the below formula (3),
$W_0$ represents a hydroxy group,
$X_0$ are the same as those defined in the below R' group.
when m is an integer of 3 or more, the nucleic acid oligomer represented by formula (4) represents a nucleic acid oligomer which may be incorporated by a non-nucleotide linker instead of the number of p (with the proviso

that p is a positive integer satisfying an equation: m-1 > p) of nucleotides between the respective nucleotides at 5' terminus and 3' terminus),
which comprises contacting a nucleic acid oligomer represented by formula (3):

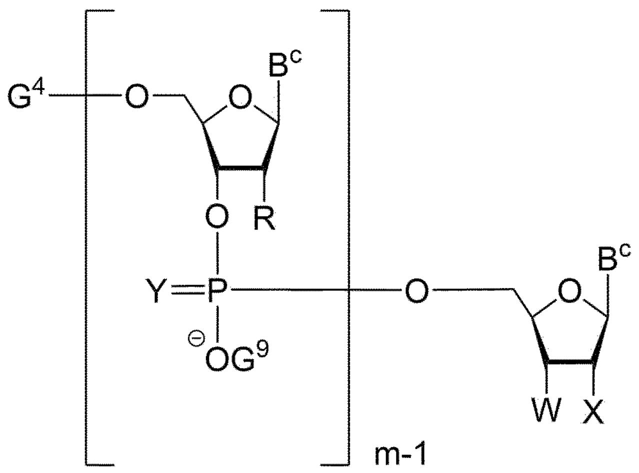

(3)

(wherein

G$^4$ represents a hydrogen atom or a protecting group of hydroxy group,
G$^9$ represents an ammonium ion, an alkylammonium ion, an alkali metal ion, a hydrogen ion, or a hydroxyalkylammonium ion,
B$^c$ represents a nucleobase, each of which is independently identical to or different from each other,
R are identical to or different from each other and each independently represents a hydrogen atom, a fluorine atom or OQ group,
Q is identical to or different from each other and each independently represents a tert-butyldimethylsilyl group, a methyl group, a 2-methoxyethyl group, a methylene group which is attached to a carbon atom at 4' position of ribose, an ethylene group which is attached to a carbon atom at 4' position of ribose, an ethylidene group which is attached to a carbon atom at 4' position of ribose, or a protecting group represented by formula (1):

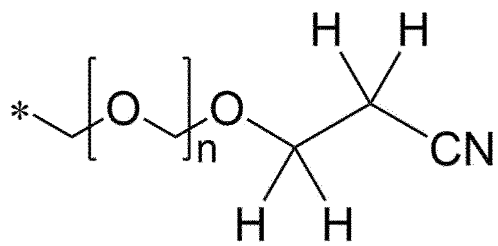

(1)

(wherein

a bond marked with * represents a bond to an oxygen atom of OQ group,
n is an integer of 0 or more),
Y are identical to or different from each other and each independently represents an oxygen atom or a sulfur atom,
m is an integer of 2 or more to 200 or less,
W and X are defined as either the following (a) or (b) :

(a) when W represents a hydroxy group, X is the same as defined as those of the above R group,
(b) when X represents a hydroxy group, W represents a OV group,

V represents a tert-butyldimethylsilyl group, or the group represented by the above formula (1).
with proviso that at least one group selected from the above R group, W group and X group represents a hydroxy group which is protected with a protecting group represented by the above formula (1), and

when m is an integer of 3 or more, the nucleic acid oligomer represented by formula (3) represents a nucleic acid oligomer which may be incorporated by a non-nucleotide linker instead of the number of p (with the proviso that p is a positive integer satisfying an equation: m-1 > p) of nucleotides between the respective nucleotides at 5' terminus and 3' terminus)

with a fluoride ion under an atmosphere of an inert gas or inert gases containing 15 % or less of oxygen concentration.

2. The process according to claim 1 wherein n is 0 or 1 in formula (1).

3. The process according to claim 1 wherein n is 0 in formula (1).

4. The process according to claim 1 wherein n is 1 in formula (1).

5. The process according to any one of claims 1 to 4 wherein the non-nucleotide linker is a linker comprising an amino acid backbone.

6. The process according to claim 5 wherein the linker comprising an amino acid backbone is a linker having a structure selected from the following formula (A14-1), (A14-2) or (A14-3).

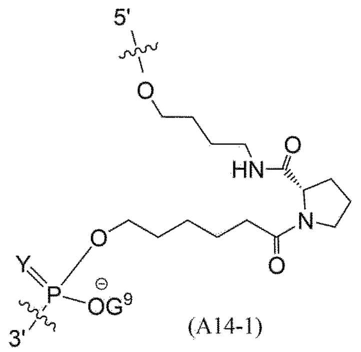

(A14-1) ,

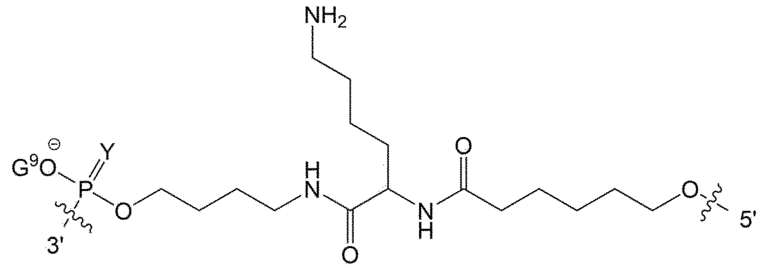

(A14-2)

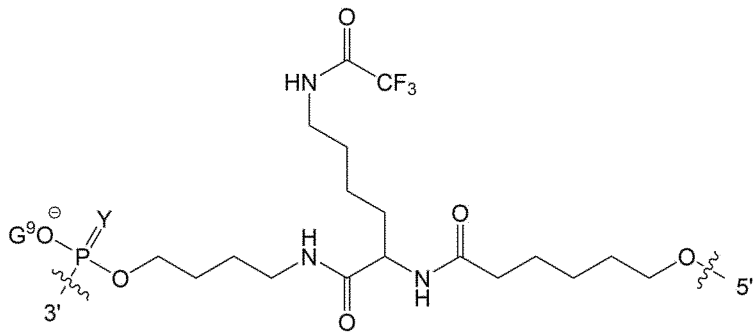

(A14-3)

(wherein a symbol of 5' and 3' represents a 5' terminus side and 3' terminus side respectively of the nucleic acid oligomer.).

7. The process according to any one of claims 1 to 5 wherein W represents a hydroxy group, X represents a R group, $W_0$ represents a hydroxy group, and $X_0$ represents a R' group.

8. The process according to any one of claims 1 to 7 wherein the fluoride ion source is tetraalkylammonium fluoride.

9. The process according to any one of claims 1 to 8 wherein the tetraalkylammonium fluoride is tetra-n-butylammonium fluoride (TBAF).

10. The process according to any one of claims 1 to 9 wherein the reaction is conducted under an atmosphere of an

inert gas or inert gases containing 10 % or less of oxygen concentration.

**11.** The process according to any one of claims 1 to 9 wherein the reaction is conducted under an atmosphere of an inert gas or inert gases containing 5 % or less of oxygen concentration.

**12.** The process according to any one of claims 1 to 9 wherein the reaction is conducted under an atmosphere of an inert gas or inert gases containing 4 % or less of oxygen concentration.

**13.** The process according to any one of claims 1 to 9 wherein the reaction is conducted under an atmosphere of an inert gas or inert gases containing 3 % or less of oxygen concentration.

**14.** The process according to any one of claims 1 to 9 wherein the reaction is conducted under an atmosphere of an inert gas or inert gases containing 2 % or less of oxygen concentration.

**15.** The process according to any one of claims 1 to 9 wherein the reaction is conducted under an atmosphere of an inert gas or inert gases containing 1 % or less of oxygen concentration.

**16.** The process according to any one of claims 1 to 9 wherein the reaction is conducted under an atmosphere of an inert gas or inert gases containing 0 % of oxygen concentration.

**17.** The process according to any one of claims 1 to 16 wherein the temperature in the reaction system in which the nucleic acid oligomer represented by formula (3) is contacted with the fluoride ion is 25 °C or less.

**18.** The process according to any one of claims 1 to 16 wherein the temperature in the reaction system in which the nucleic acid oligomer represented by formula (3) is contacted with the fluoride ion is 20 °C or less.

**19.** The process according to any one of claims 1 to 16 wherein the temperature in the reaction system in which the nucleic acid oligomer represented by formula (3) is contacted with the fluoride ion is 15 °C or less.

**20.** The process according to any one of claims 1 to 16 wherein the temperature in the reaction system in which the nucleic acid oligomer represented by formula (3) is contacted with the fluoride ion is 10 °C or less.

**21.** The process according to any one of claims 1 to 16 wherein the temperature in the reaction system in which the nucleic acid oligomer represented by formula (3) is contacted with the fluoride ion is 5 °C or less.

**22.** The process according to any one of claims 1 to 16 wherein a duration required for contacting the total amount of the fluoride ion with the nucleic acid oligomer represented by formula (3) is 30 minutes or more.

**23.** The process according to any one of claims 1 to 16 wherein a duration required for contacting the total amount of the fluoride ion with the nucleic acid oligomer represented by formula (3) is 1 hour or more.

**24.** The process according to any one of claims 1 to 23 wherein among the R group, the W group, and the X group in the nucleic acid oligomer represented by formula (3), the ratio of a protecting group represented by the above formula (1) is 10 % or more, and the nucleic acid chain length is 10 mer or more.

**25.** The process according to any one of claims 1 to 23 wherein among the R group, the W group, and the X group in the nucleic acid oligomer represented by formula (3), the ratio of a protecting group represented by the above formula (1) is 20 % or more, and the nucleic acid chain length is 10 mer or more.

**26.** The process according to any one of claims 1 to 23 wherein among the R group, the W group, and the X group in the nucleic acid oligomer represented by formula (3), the ratio of a protecting group represented by the above formula (1) is 30 % or more, and the nucleic acid chain length is 10 mer or more.

**27.** The process according to any one of claims 1 to 23 wherein among the R group, the W group, and the X group in the nucleic acid oligomer represented by formula (3), the ratio of a protecting group represented by the above formula (1) is 40 % or more, and the nucleic acid chain length is 10 mer or more.

**28.** The process according to any one of claims 1 to 23 wherein among the R group, the W group, and the X group in

the nucleic acid oligomer represented by formula (3), the ratio of a protecting group represented by the above formula (1) is 50 % or more, and the nucleic acid chain length is 10 mer or more.

**29.** The process according to any one of claims 1 to 23 wherein among the R group, the W group, and the X group in the nucleic acid oligomer represented by formula (3), the ratio of a protecting group represented by the above formula (1) is 60 % or more, and the nucleic acid chain length is 10 mer or more.

**30.** The process according to any one of claims 1 to 23 wherein among the R group, the W group, and the X group in the nucleic acid oligomer represented by formula (3), the ratio of a protecting group represented by the above formula (1) is 70 % or more, and the nucleic acid chain length is 10 mer or more.

**31.** The process according to any one of claims 1 to 23 wherein among the R group, the W group, and the X group in the nucleic acid oligomer represented by formula (3), the ratio of a protecting group represented by the above formula (1) is 80 % or more, and the nucleic acid chain length is 10 mer or more.

**32.** The process according to any one of claims 1 to 23 wherein among the R group, the W group, and the X group in the nucleic acid oligomer represented by formula (3), the ratio of a protecting group represented by the above formula (1) is 90 % or more, and the nucleic acid chain length is 10 mer or more.

**33.** The process according to any one of claims 1 to 23 wherein among the R group, the W group, and the X group in the nucleic acid oligomer represented by formula (3), the ratio of a protecting group represented by the above formula (1) is 95 % or more, and the nucleic acid chain length is 10 mer or more.

**34.** The process according to any one of claims 1 to 23 wherein among the R group, the W group, and the X group in the nucleic acid oligomer represented by formula (3), the ratio of a protecting group represented by the above formula (1) is 100 %, and the nucleic acid chain length is 10 mer or more.

**35.** The process according to any one of claims 1 to 34 wherein the nucleic acid chain length is 20 mer or more.

**36.** The process according to any one of claims 1 to 34 wherein the nucleic acid chain length is 30 mer or more.

**37.** The process according to any one of claims 1 to 34 wherein the nucleic acid chain length is 40 mer or more.

**38.** The process according to any one of claims 1 to 34 wherein the nucleic acid chain length is 50 mer or more.

## INTERNATIONAL SEARCH REPORT

International application No.
**PCT/JP2021/030423**

**A. CLASSIFICATION OF SUBJECT MATTER**

***C07H 21/02***(2006.01)i; ***C12N 15/09***(2006.01)i
FI: C07H21/02; C12N15/09 Z ZNA

According to International Patent Classification (IPC) or to both national classification and IPC

**B. FIELDS SEARCHED**

Minimum documentation searched (classification system followed by classification symbols)

C07H21/02; C12N15/09

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | 公益社団法人 日本化学会, 化学便覧 応用化学編, seventh edition, 丸善出版株式会社, 2014, pp. 504, 505, non-official translation (THE CHEMICAL SOCIETY OF JAPAN, Chemistry Handbook, Applied Chemistry, MARUZEN PUBLISHING CO., LTD.) fig. 8, 65, page 504, left column, line 7 to page 505, left column, line 12 | 1-3, 7-16, 22-38 |
| Y | | 1-38 |
| Y | JP 60-105692 A (NIPPON ZEON KK) 11 June 1985 (1985-06-11) claims, page 1, right column, lines 6-11 | 1-38 |
| Y | WO 2013/027843 A1 (BONAC CORP.) 28 February 2013 (2013-02-28) examples | 4-38 |
| A | | 1-3 |
| Y | JP 5555346 B2 (BONAC CORP.) 23 July 2014 (2014-07-23) claims, examples | 5-38 |
| Y | JP 5876890 B2 (BONAC CORP.) 02 March 2016 (2016-03-02) claims, examples | 5-38 |
| A | | 1-4 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 October 2021** | **26 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/030423**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/030423**

| Patent document cited in search report | Publication date (day/month/year) | Patent family member(s) | Publication date (day/month/year) |
|---|---|---|---|
| JP 60-105692 A | 11 June 1985 | (Family: none) | |
| WO 2013/027843 A1 | 28 February 2013 | US 2014/0206856 A1 examples<br>US 2016/0176810 A1<br>EP 2749565 A1<br>CA 2846572 A1<br>CN 103906758 A<br>KR 10-2014-0067062 A | |
| JP 5555346 B2 | 23 July 2014 | US 2012/0010271 A1 examples<br>US 2012/0035246 A1<br>US 2014/0171486 A1<br>US 2014/0171633 A1<br>US 2015/0011605 A1<br>US 2016/0108400 A1<br>US 2015/0249031 A1<br>US 2016/0132045 A1<br>US 2016/0358795 A1<br>US 2019/0109032 A1<br>US 2020/0176289 A1<br>US 2018/0240691 A1<br>EP 2431466 A1<br>EP 2436767 A1<br>EP 2628801 A1<br>EP 2639307 A2<br>EP 2674494 A1<br>EP 2933333 A1<br>CN 102918156 A<br>CA 2804511 A1<br>CN 103052711 A<br>KR 10-2013-0090792 A<br>KR 10-2013-0095738 A<br>CN 104004018 A<br>CN 104059911 A<br>CN 104818276 A<br>KR 10-2018-0040728 A<br>KR 10-2018-0040727 A | |
| JP 5876890 B2 | 02 March 2016 | US 2014/0329886 A1 examples<br>WO 2013/103146 A1<br>EP 2801617 A1<br>CN 104039961 A<br>KR 10-2014-0111688 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020159460 A
- WO 2006022323 A1
- WO 2013027843 A1
- JP 5157168 B
- JP 5554881 B
- JP 5555346 B
- JP 5876890 B
- WO 2012005368 A1
- WO 2018182008 A
- WO 2019074110 A
- JP 3745226 B
- WO 2001053528 A1
- JP 2014221817 A
- JP 4705716 B
- WO 2019060442 A
- JP 4965745 B
- JP 2015523856 A
- JP 2017537626 A
- US 20120035246 A
- WO 2017188042 A

### Non-patent literature cited in the description

- **XIULONG, SHEN et al.** *Nucleic Acids Research,* 2018, vol. 46 (46), 1584-1600
- **DANIEL O'REILLY et al.** *Nucleic Acids Research,* 2019, vol. 47 (2), 546-558
- *Nucleic Acids Research,* 2019, vol. 47 (2), 547